# EUROPÄISCHE PATENTSCHRIFT

(11) **EP 3 250 265 B1**
(45) Veröffentlichungstag und Bekanntmachung des Hinweises auf die Patenterteilung: **09.01.2019**
(21) Anmeldenummer: 15808081.2
(22) Anmeldetag: 27.11.2015
(51) Int. Cl.: A61M 5/24, A61M 5/32, A61M 5/145, G01F 25/00, G01F 11/02, G01F 23/26, G01F 22/00

(54) **ABGABEVORRICHTUNG ZUR ABGABE VON FLÜSSIGKEITEN UND VERFAHREN ZUR BESTIMMUNG EINES PARAMETERS EINER FLÜSSIGKEIT IN EINEM BEHÄLTER**
LIQUID DISPENSING DEVICE AND METHOD OF DETERMINING A PARAMETER OF A LIQUID IN A CONTAINER
DISPENSATEUR DE LIQUIDES ET PROCÉDÉ POUR DÉTERMINER UN PARAMÈTRE D'UN LIQUIDE DANS UN RÉCIPIENT

(30) Priorität: 28.01.2015 AT 500582015
(43) Veröffentlichungstag der Anmeldung: 06.12.2017
(73) Patentinhaber: AIT Austrian Institute of Technology GmbH, 1220 Wien (AT); Seibersdorf Labor GmbH, 2444 Seibersdorf (AT)
(72) Erfinder: SCHMID, Gernot, 2833 Bromberg (AT); BAMMER, Manfred, 1220 Wien (AT); HIRTL, Rene, 1200 Wien (AT); LURF, Robert, 2640 Gloggnitz (AT); MEINDL, Michael, 2632 Wimpassing (AT); BEISTEINER, Martin, 2853 Bad Schönau (AT)
(74) Vertreter: Wildhack & Jellinek
(86) Internationale Anmeldenummer: PCT/AT2015/050302
(87) Internationale Veröffentlichungsnummer: WO 2016/118987

(56) Entgegenhaltungen:
- EP-A1- 2 361 647
- WO-A1-2014/052997
- US-A- 5 720 733

## Beschreibung

Die Erfindung betrifft eine Abgabevorrichtung zur Abgabe von Flüssigkeiten, insbesondere flüssigen Medikamenten gemäß dem Oberbegriff des Patentanspruchs 1. Weiters betrifft die Erfindung ein Verfahren zur Bestimmung eines im Zusammenhang mit einem Behälter oder einer in dem Behälter befindlichen Flüssigkeit stehenden physikalischen oder chemischen Parameters, insbesondere des Füllstands der Flüssigkeit im Behälter gemäß dem Oberbegriff des Patentanspruchs 18.

Aus dem Stand der Technik sind unterschiedliche Abgabevorrichtungen und Verfahren zur Bestimmung von Füllständen in Abgabevorrichtungen oder anderen Parametern, die mit der Flüssigkeit im Behälter in Beziehung stehen, bekannt, die auf kapazitiver Detektion basieren (siehe zum Beispiel US 5 720 733 A und WO 2014/052997 A1). Wesentlicher Nachteil all dieser Verfahren bzw. Abgabevorrichtungen ist, dass die Verwendung einer leitfähigen bzw. metallischen Injektionsnadel zur Verabreichung der jeweiligen Flüssigkeit die auf kapazitiven Messprinzipien beruhenden Messungenbeeinflussen, sodass abhängig davon, ob an der Abgabevorrichtung eine Injektionsnadel aufgesetzt ist oder nicht, unterschiedliche Messergebnisse vorliegen.

Der Erfindung liegt die Aufgabe zugrunde, eine Abgabevorrichtung sowie ein Verfahren zu schaffen, das diese Nachteile überwindet.

EP 2 361 647 A1 offenbart eine Nadeldetektionsschaltung zur Erkennung, ob eine Injektionsnadel aufgesetzt ist oder nicht.

Die Erfindung löst die Aufgabe bei einer Abgabevorrichtung der eingangs genannten Art mit den kennzeichnenden Merkmalen der Patentansprüche 1 bzw. 18.

Die Erfindung gemäss Anspruch 1 sieht bei einer Abgabevorrichtung zur Abgabe von Flüssigkeiten, insbesondere von flüssigen

### Medikamenten an Personen, umfassend

- einen mit der Flüssigkeit gefüllten Behälter, der an einem Ende eine Öffnung zur Abgabe der Flüssigkeit aufweist, wobei vorgesehen ist, dass im Bereich der Öffnung Haltemittel zum Halten elektrisch leitfähigen Injektionsnadel zum Injizieren der im Behälter befindlichen Flüssigkeit vorhanden sind,
- ein Messsystem zur kapazitiven Bestimmung eines Messwerts, sowie
- eine Verarbeitungseinheit zur Bestimmung zumindest eines in Zusammenhang mit dem Behälter oder der darin befindlichen Flüssigkeit stehenden physikalischen oder chemischen Parameterwert, insbesondere des Füllstands, aufgrund des Messwerts vor,
- dass zumindest ein Detektionselement im Bereich der Öffnung, vorhanden ist,
- dass eine Nadeldetektionsschaltung vorhanden ist, wobei das Detektionselement an die Nadeldetektionsschaltung angeschlossen ist,
- dass die Nadeldetektionsschaltung zur Messung einer am Ausgang des Detektionselements abgreifbaren elektrischen Kenngröße, insbesondere der Induktivität, Kapazität oder Leitfähigkeit, ausgebildet ist,
- dass eine Verarbeitungseinheit vorhanden ist, und das am Ausgang der Nadeldetektionsschaltung anliegende Messergebnis als Detektionsergebnis der Verarbeitungseinheit zugeführt ist, und
- dass die Verarbeitungseinheit den Parameter in Abhängigkeit des Messwertes und des Detektionsergebnisses berechnet und an ihrem Ausgang zur Verfügung hält.

Hierbei wird vorteilhaft erreicht, dass die elektrische Einflussnahme einer metallischen oder leitfähigen Injektionsnadel auf das Messergebnis weitestgehend eliminiert wird.

Eine Möglichkeit, den Einfluss einer metallischen oder leitfähigen Injektionsnadel auf das Messergebnis zu kompensieren, sieht vor, dass die Verarbeitungseinheit das ihr zugeführte Detektionsergebnis mit einem vorgegebenen Schwellenwert vergleicht, und den am Ausgang zur Verfügung gehaltene Parameterwert auf Basis des Messwertes berechnet, wobei sie die zur Berechnung des Parameterwert verwendete Berechnungsvorschrift abhängig vom Ergebnis des Vergleichs des Detektionsergebnisses mit dem vorgegebenen Schwellenwert ermittelt.

Ein einfaches Vorgehen zur numerischen Beschreibung und Bereinigung der Einflussnahme einer metallischen oder leitfähigen Injektionsnadel auf das Messergebnis sieht vor,
- dass in der Verarbeitungseinheit eine erste Kalibrierfunktion abgespeichert ist, die den Zusammenhang zwischen dem Messwert und dem mit dem Behälter oder der darin befindlichen Flüssigkeit in Zusammenhang stehenden physikalischen oder chemischen Parameterwert bei Vorhandensein einer Injektionsnadel im Haltemittel angibt,
- dass in der Verarbeitungseinheit eine zweite Kalibrierfunktion abgespeichert ist, die den Zusammenhang zwischen dem Messwert und dem mit dem Behälter oder der darin befindlichen Flüssigkeit in Zusammenhang stehenden physikalischen oder chemischen Parameterwert bei Fehlen einer Injektionsnadel im Haltemittel angibt, und
- dass die Verarbeitungseinheit die für die Berechnung des Parameters verwendete Kalibrierfunktion in Abhängigkeit des Vergleichs des Detektionsergebnisses mit dem vorgegebenen Schwellenwert auswählt.

### Alternativ dazu kann vorgesehen sein,

- dass die Verarbeitungseinheit eine erste Einheit und eine zweite Einheit umfasst,
- dass in der ersten Einheit eine erste Kalibrierfunktion abgespeichert ist, die den Zusammenhang zwischen dem Messwert und dem mit dem Behälter oder der darin befindlichen Flüssigkeit in Zusammenhang stehenden physikalischen oder chemischen Parameterwert bei Vorhandensein einer Injektionsnadel im Haltemittel angibt, wobei die erste Einheit die erste Kalibrierfunktion auf den bei ihr anliegenden Messwert anwendet und das so erhaltene Ergebnis an ihrem Ausgang als ersten Parameterwert zur Verfügung hält, und
- dass in der zweiten Einheit eine zweite Kalibrierfunktion abgespeichert ist, die den Zusammenhang zwischen dem Messwert und dem mit dem Behälter oder der darin befindlichen Flüssigkeit in Zusammenhang stehenden physikalischen oder chemischen Parameterwert bei Fehlen einer Injektionsnadel im Haltemittel angibt, wobei die zweite Einheit die zweite Kalibrierfunktion auf den bei ihr anliegenden Messwert anwendet und das so erhaltene Ergebnis an ihrem Ausgang als zweiten Parameterwert zur Verfügung hält.

### Dabei ist vorteilhaft vorgesehen,

- dass die Verarbeitungseinheit eine erste Einheit zur Bestimmung eines ersten Parameterwertes aufweist, die eine erste Berechnungsvorschrift, die aufgrund des Messwerts den Parameterwert bei Vorhandensein der Injektionsnadel in den Haltemitteln liefert, auf den Messwert anwendet,
- dass die Verarbeitungseinheit eine zweite Einheit zur Bestimmung eines zweiten Parameterwertes aufweist, die eine zweite Berechnungsvorschrift, die aufgrund des Messwerts den Parameter bei Fehlen der Injektionsnadel in den Haltemitteln liefert, auf den Messwert anwendet, und
- dass die Verarbeitungseinheit das ihr zugeführte Detektionsergebnis mit einem vorgegebenen Schwellenwert vergleicht und abhängig von diesem Vergleich entweder den von der ersten Einheit oder den von der zweiten Einheit ermittelten ersten oder zweiten Parameter als Parameter an ihrem Ausgang zur Verfügung hält.

Eine besonders einfache und vorteilhafte Kompensation des kapazitiven Einflusses der Injektionsnadel auf die vorteilhafte kapazitive Bestimmung von Messwerten kann vorsehen, dass an das Messsystem zumindest ein Paar von im Außenbereich, insbesondere anliegend an der Wand des Behälters angeordneten Messelektroden zur Bestimmung der zwischen den Messelektroden abgreifbaren Kapazität angeschlossen sind und dieser Kapazitätsmesswert als Messwert der Verarbeitungseinheit zugeführt ist. Um eine Verfälschung des Messergebnisses durch externe Felder oder Berührungen der Messelektroden durch den Messenden zu vermeiden, kann vorgesehen sein, dass insbesondere zumindest eine die Messelektroden umgebende weitere Metallstruktur vorhanden ist, und vorzugsweise zumindest eine der Metallstrukturen als elektrische Abschirmung ausgebildet ist.

Um eine rasche und sichere Detektion des Vorhandenseins oder Fehlens einer Injektionsnadel bei gleichzeitig einfachem mechanischem Aufbau zu gewährleisten, kann vorgesehen sein,
- dass die aufsteckbare Kappe den auf der Seite der Öffnung befindlichen Bereich der Abgabevorrichtung zumindest teilweise und eine allenfalls von dem Haltemittel gehaltene Injektionsnadel vollständig umgibt oder die eine Ausnehmung aufweist, durch die eine allenfalls von dem Haltemittel gehaltene Injektionsnadel hindurch ragt,
- dass das Detektionselement auf oder in der aufsteckbaren Kappe im Bereich des Haltemittels oder im Bereich der Injektionsnadel angeordnet ist, und
dass insbesondere das Messsystem und die Nadeldetektionsschaltung in der aufsteckbaren Kappe integriert sind.

Bevorzugte Ausführungsformen, bei denen die Detektion der Injektionsnadel durch eine Kapazitätsmessung erfolgt sehen vor,
- dass das Detektionselement als leitfähige im Bereich der Öffnung angeordnete Detektionselektrode ausgebildet ist,
- dass die Detektionselektrode elektrisch an eine Nadeldetektionsschaltung gekoppelt ist.

Insbesondere kann dabei vorgesehen sein, dass als metallische Struktur zumindest eine der Messelektroden des Messsystems fungiert.

Eine besonders bevorzugte Möglichkeit, das Vorhandensein einer Injektionsnadel durch Kapazitätsmessung zu detektieren, sieht vor,
- dass die Detektionselektrode und gegebenenfalls die metallische Struktur, insbesondere in Form einer weiteren Detektionselektrode, in oder auf der aufsteckbaren Kappe im Bereich des Haltemittels oder im Bereich der Injektionsnadel angeordnet sind, und/oder
- dass die Detektionselektrode sowie gegebenenfalls die metallische Struktur, insbesondere in Form einer weiteren Detektionselektrode, im Bereich der Haltemittel für die Injektionsnadel an der Außenwand des Behälters angeordnet sind, und/oder
- dass die Detektionselektrode sowie gegebenenfalls die metallische Struktur, insbesondere in Form einer weiteren Detektionselektrode, im Bereich der Haltemittel für die Injektionsnadel im Außenbereich der Abgabevorrichtung, beabstandet vom Behälter angeordnet sind.

Eine weitere einfache Detektion einer Injektionsnadel sieht vor
- dass das Detektionselement durch einen Schalter gebildet wird,
- dass die Nadeldetektionsschaltung zur Messung der elektrischen Leitfähigkeit zwischen den Kontakten des Schalters ausgebildet ist,
- dass der Schalter durch das Vorhandensein einer Injektionsnadel mechanisch betätigt wird, und
- dass der Schalter an die Verarbeitungseinheit elektrisch gekoppelt ist.

Hierbei ist es zur Detektion von Vorteil, wenn aufgrund der Betätigung des Schalters dem Schwellwertvergleicher der Verarbeitungseinheit über den Schalter ein elektrisches Signal als Detektionsergebnis zugeführt ist.

Alternativ lässt sich das Vorhandensein einer Injektionsnadel auch induktiv bestimmen, insbesondere indem
- dass das Detektionselement durch eine Leiteranordnung oder Spule, gebildet wird, deren Induktivität bei Vorhandensein einer Injektionsnadel einen anderen Wert aufweist, als bei Fehlen der Injektionsnadel,
- dass die Leiteranordnung oder Spule elektrisch an die als eine Induktivitätsmessschaltung ausgebildete Nadeldetektionsschaltung gekoppelt ist,
- dass die Nadeldetektionsschaltung zur Messung der Induktivität der Leiteranordnung oder Spule vorhanden ist, wobei das am Ausgang der Nadeldetektionsschaltung anliegende Detektionsergebnis in Form eines Induktivitätsmessergebnisses der Verarbeitungseinheit zugeführt ist,
- dass die Verarbeitungseinheit den Parameter in Abhängigkeit des Messergebnisses und des Detektionsergebnisses berechnet und an ihrem Ausgang zur Verfügung hält, wobei insbesondere
- die Leiteranordnung oder Spule im Bereich der Haltemittel für die Injektionsnadel an der Außenwand des Behälters angeordnet sind, oder
- dass die Leiteranordnung oder Spule im Bereich der Haltemittel für die Injektionsnadel im Außenbereich der Abgabevorrichtung, beabstandet vom Behälter angeordnet sind.

Vorteilhafte Anordnungen der Spulen sehen vor, dass die Spule derart angeordnet ist, dass bei Vorhandensein der Injektionsnadel diese von den Windungen der Spule zumindest teilweise umschlungen wird.

Dabei kann auch vorgesehen sein, dass die Leiteranordnung oder Spule in oder auf der aufsteckbaren Kappe im Bereich des Haltemittels oder im Bereich der Injektionsnadel angeordnet sind, wobei insbesondere die Spule derart angeordnet ist, dass bei Vorhandensein der Injektionsnadel und auf die Abgabevorrichtung aufgesteckter Kappe, die Injektionsnadel von den Windungen der Spule zumindest teilweise umschlungen wird. Weiters kann das Vorhandensein einer Injektionsnadel bestimmt werden, indem das Detektionselement durch zwei Kontaktelemente gebildet wird, die bei Vorhandensein einer elektrisch leitfähigen Injektionsnadel mit dieser in elektrischem Kontakt stehen, wobei insbesondere über die elektrische Verbindung zwischen den Kontaktelementen und der elektrisch leitfähigen Nadel dem Schwellwertvergleicher der Verarbeitungseinheit ein elektrisches Signal als Detektionsergebnis zugeführt wird.

Von besonderem hygienischen Vorteil bei der Detektion der Injektionsnadel ist es, wenn
- auf einem mit der Injektionsnadel unlösbar verbundenem Halteteil eine elektrisch leitfähige Beschichtung vorhanden ist, und
- zwei Detektionselemente in Form von Kontaktelemente vorgesehen sind, die bei Vorhandensein einer Injektionsnadel mit der elektrisch leitfähigen Beschichtung in elektrischem Kontakt stehen, wobei insbesondere
- über die elektrische Verbindung zwischen den Kontaktelementen und der elektrisch leitfähigen Beschichtung dem Schwellwertvergleicher der Verarbeitungseinheit ein elektrisches Signal als Detektionsergebnis zugeführt wird, und/oder
- die Kontaktelemente in oder auf der aufsteckbaren Kappe im Bereich des Haltemittels oder im Bereich der Injektionsnadel angeordnet sind.

Weiters ist erfindungsgemäß bei einem Verfahren zur Bestimmung eines in Zusammenhang mit einem Behälter oder einer in dem Behälter befindlichen Flüssigkeit stehenden physikalischen oder chemischen Parameters, insbesondere des Füllstands der Flüssigkeit im Behälter,
- wobei der Behälter an einem Ende eine Öffnung zur Abgabe der Flüssigkeit aufweist, und wobei im Bereich der Öffnung Haltemittel zum Halten einer elektrisch leitfähigen Injektionsnadel zum Injizieren der im Behälter befindlichen Flüssigkeit vorhanden sind, und
- wobei ein Messwert im Bereich des Behälters ermittelt wird, vorgesehen,
- dass mittels eines im Bereich der Öffnung befindlichen Detektionselementes ein vom Vorhandensein einer Injektionsnadel abhängiges Detektionsergebnis, ermittelt wird, durch Ermittlung der Kapazität zwischen einer im Bereich der Öffnung befindlichen metallischen Detektionselektrode und einer in oder auf der Abgabevorrichtung angeordneten weiteren metallischen Struktur,
- dass das Detektionsergebnis mit einem Schwellenwert verglichen wird und abhängig vom Schwellenwertvergleich das Vorhandensein oder das Fehlen einer Injektionsnadel im Haltemittel festgestellt wird, wobei
- bei Detektion des Vorhandenseins einer Injektionsnadel ein erster Parameter durch Anwendung einer ersten Berechnungsvorschrift, die aufgrund des Messwerts den Parameterwert bei Vorhandensein der Injektionsnadel in den Haltemitteln liefert, auf den Messwert angewendet wird, und der so ermittelte erste Parameterwert als Parameterwert zur Verfügung gehalten wird, und
- bei Detektion des Fehlens einer Injektionsnadel ein zweiter Parameterwert durch Anwendung einer zweiten Berechnungsvorschrift, die aufgrund des Messwerts den Parameterwert bei Fehlen der Injektionsnadel in den Haltemitteln liefert, auf den Messwert angewendet wird, und der so ermittelte zweite Parameter als Parameterwert zur Verfügung gehalten wird.

Hierdurch wird auf einfache Weise ein Verfahren zur Verfügung gestellt, mit dem ein physikalischer Parameter, der mit der im Behälter befindlichen Flüssigkeit in Zusammenhang steht, ermittelt werden kann, ohne dass dieser durch das Vorhandensein oder Fehlen der Injektionsnadel nachteilig beeinflusst würde.

Ein einfaches Vorgehen zur numerischen Beschreibung und Bereinigung der Einflussnahme einer metallischen oder leitfähigen oder magnetisch aktiven Injektionsnadel auf das Messergebnis sieht vor,
- dass eine erste Kalibrierfunktion vorgegeben wird, die den Zusammenhang zwischen dem Messwert und dem mit dem Behälter oder der darin befindlichen Flüssigkeit in Zusammenhang stehenden physikalischen oder chemischen Parameterwert bei Vorhandensein einer Injektionsnadel im Haltemittel angibt,
- dass eine zweite Kalibrierfunktion vorgegeben wird, die den Zusammenhang zwischen dem Messwert und dem mit dem Behälter oder der darin befindlichen Flüssigkeit in Zusammenhang stehenden physikalischen oder chemischen Parameterwert bei Fehlen einer Injektionsnadel im Haltemittel angibt, und
- dass bei Detektion des Vorhandenseins der einer Injektionsnadel die erste Kalibrierfunktion auf den Messwert angewendet wird und das so erhaltene Ergebnis als Parameter zur Verfügung gehalten wird, und
- dass bei Detektion des Fehlens der einer Injektionsnadel die zweite Kalibrierfunktion auf den Messwert angewendet wird und das so erhaltene Ergebnis als Parameterwert zur Verfügung gehalten wird.

Um einen elektrisch besonders einfach zu detektierenden Wert zu erhalten, kann vorgesehen sein, dass als Messwert die zwischen zwei im Außenbereich, insbesondere anliegend an der Wand des Behälters, angeordneten Messelektroden, abgreifbare Kapazität herangezogen wird.

Mehrere bevorzugte Ausführungsbeispiele der Erfindung sind anhand der folgenden Zeichnungsfiguren näher dargestellt:

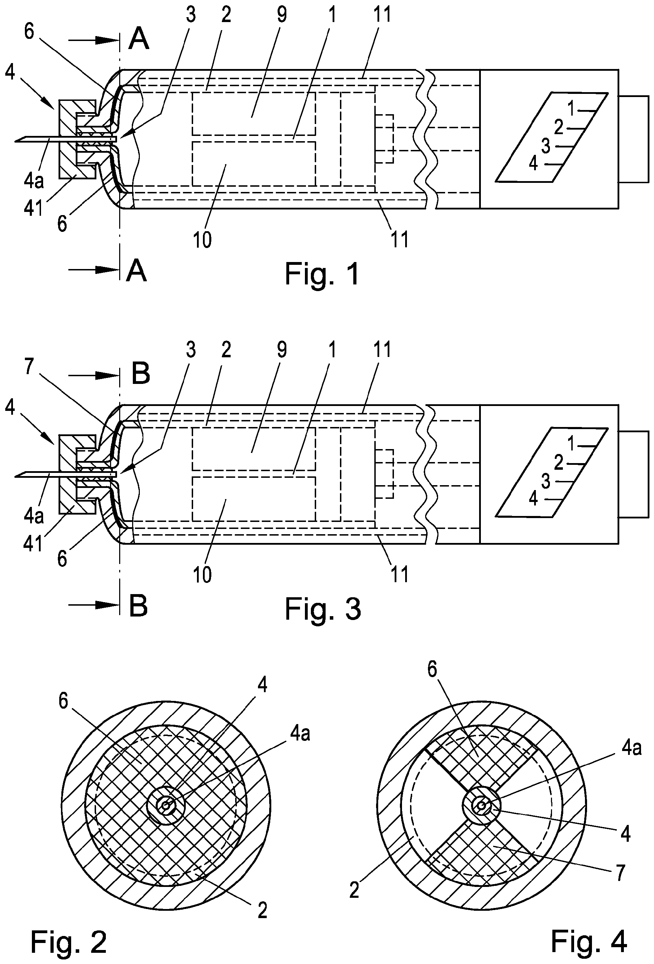

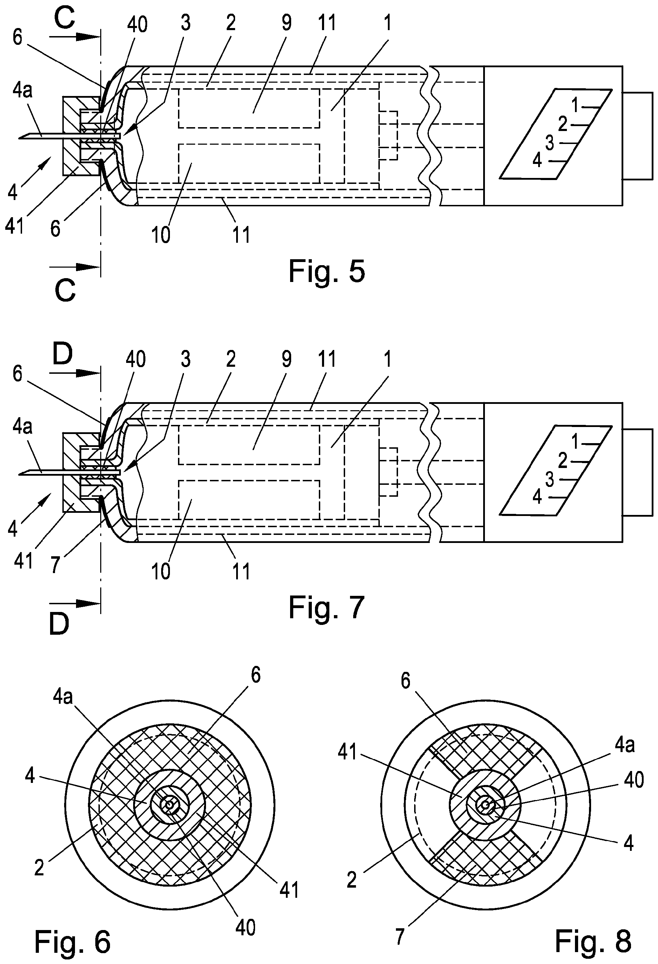

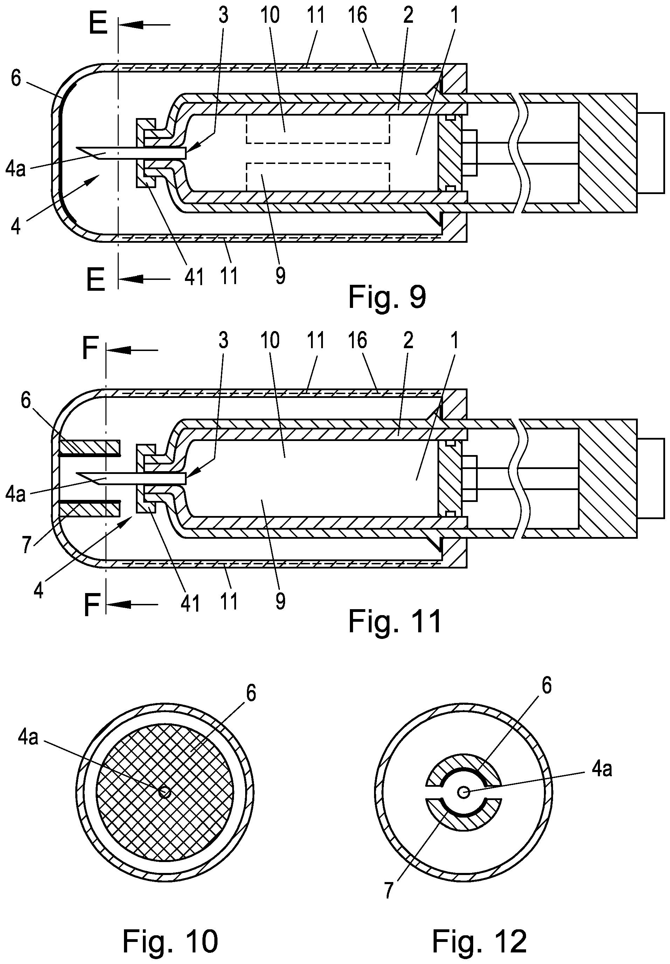

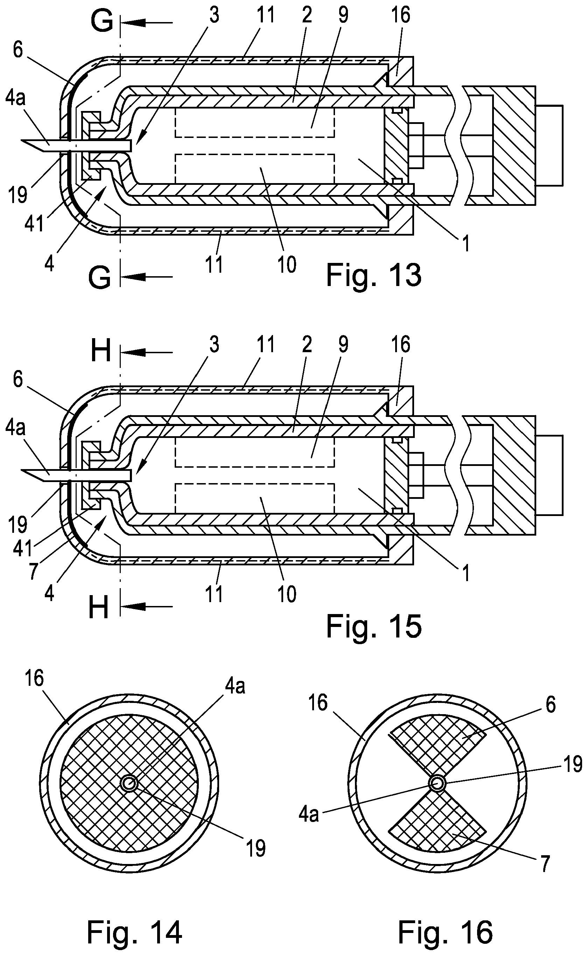

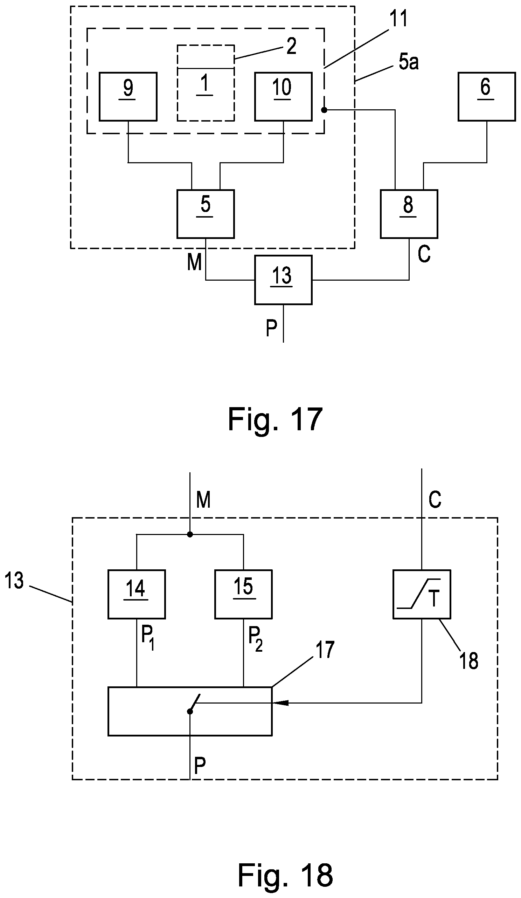

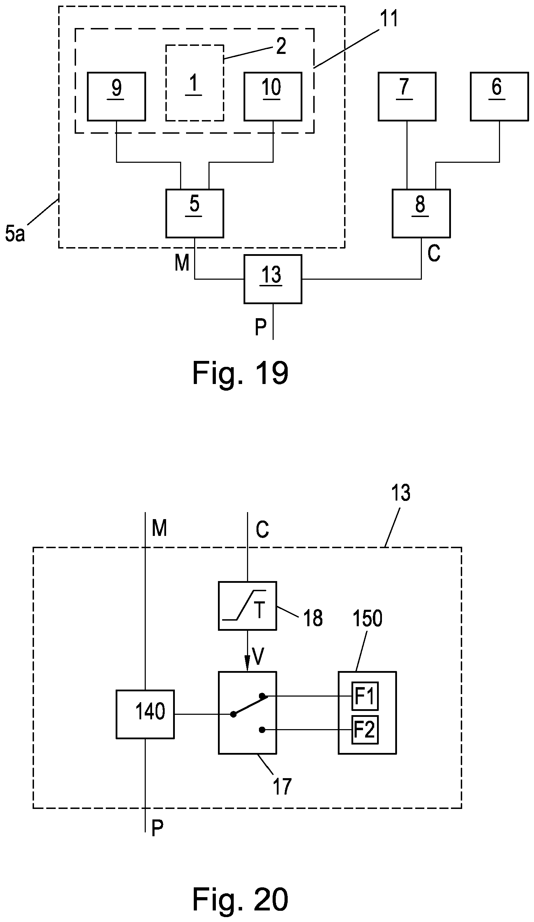

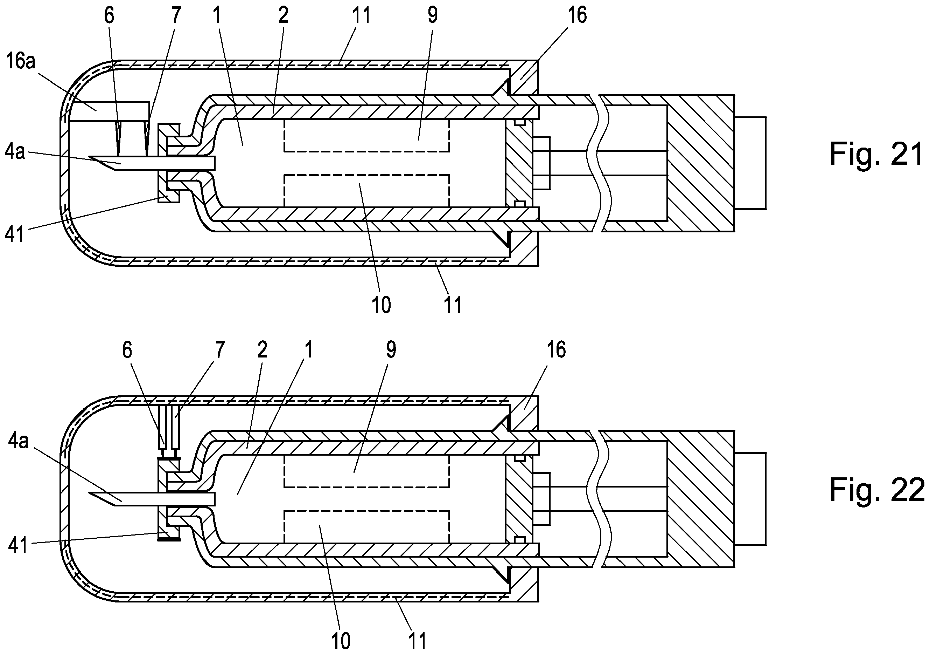

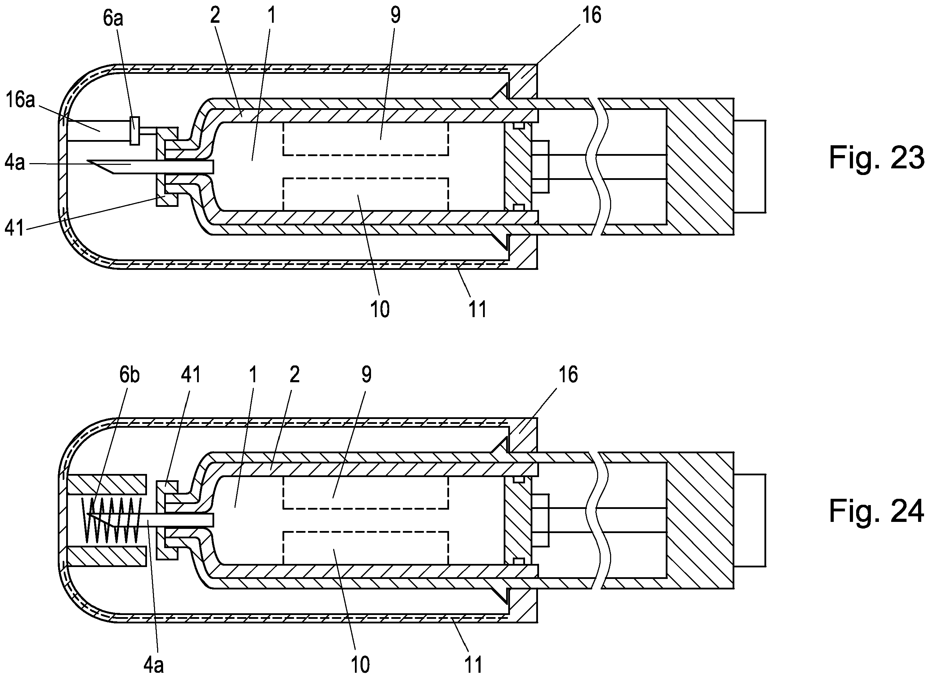

**Fig. 1** zeigt eine erste Ausführungsform einer erfindungsgemäßen Abgabevorrichtung von der Seite. **Fig. 2** zeigt einen Schnitt durch die in **Fig. 1** dargestellte Ausführungsform der Erfindung entlang der Schnittkante **A-A.** **Fig. 3** zeigt eine Abgabevorrichtung gemäß einer zweiten Ausführungsform der Erfindung. **Fig. 4** zeigt einen Schnitt durch die in **Fig. 3** dargestellte Ausführungsform der Erfindung entlang der Schnittkante **B-B.** **Fig. 5** zeigt eine dritte Ausführungsform einer erfindungsgemäßen Abgabevorrichtung von der Seite. **Fig. 6** zeigt einen Schnitt durch die in **Fig. 5** dargestellte Ausführungsform der Erfindung entlang der Schnittkante **C-C.** **Fig. 7** zeigt eine vierte Ausführungsform einer erfindungsgemäßen Abgabevorrichtung von der Seite. **Fig.** 8 zeigt einen Schnitt durch die in **Fig. 7** dargestellte Ausführungsform der Erfindung entlang der Schnittkante **D-D.** **Fig. 9** zeigt eine fünfte Ausführungsform einer erfindungsgemäßen Abgabevorrichtung von der Seite. **Fig. 10** zeigt einen Schnitt durch die in **Fig. 9** dargestellte Ausführungsform der Erfindung entlang der Schnittkante **E-E.** **Fig. 11** zeigt eine sechste Ausführungsform einer erfindungsgemäßen Abgabevorrichtung von der Seite. **Fig. 12** zeigt einen Schnitt durch die in **Fig. 11** dargestellte Ausführungsform der Erfindung entlang der Schnittkante **F-F.** **Fig. 13** zeigt eine siebente Ausführungsform einer erfindungsgemäßen Abgabevorrichtung von der Seite. **Fig. 14** zeigt einen Schnitt durch die in **Fig. 13** dargestellte Ausführungsform der Erfindung entlang der Schnittlinie **G-G.** **Fig. 15** zeigt eine achte Ausführungsform einer erfindungsgemäßen Abgabevorrichtung von der Seite. **Fig. 16** zeigt einen Schnitt durch die in **Fig. 15** dargestellte Ausführungsform der Erfindung entlang der Schnittkante **H-H.** **Fig. 17** zeigt schematisch die Verschaltung des Messsystems der Verarbeitungseinheit und der Messschaltung. **Fig. 18** zeigt die in **Fig. 17** dargestellte Verarbeitungseinheit im Detail. **Fig. 19** zeigt schematisch eine alternative Verschaltung des Messsystems der Verarbeitungseinheit und der Messschaltung. **Fig. 20** zeigt eine alternative Ausführungsform der Verarbeitungseinheit. **Fig. 21 bis 23** zeigen alternative Ausführungsformen, bei denen das Vorhandensein einer Injektionsnadel durch Änderung einer Leitfähigkeit detektierbar ist. **Fig. 24 bis 26** zeigen weitere alternative Ausführungsformen mit induktiver Detektion.

In **Fig.** 1 ist eine **erste Ausführungsform** einer erfindungsgemäßen Abgabevorrichtung zur Abgabe von Flüssigkeiten 1 dargestellt. Diese Flüssigkeit 1 befindet sich in einem Behälter 2, der an einem Ende eine Öffnung 3 zur Abgabe der Flüssigkeit aufweist. Es ist dabei vorgesehen, dass im Bereich der Öffnung 3 Haltemittel 4 zum Halten einer Injektionsnadel 4a vorgesehen sind. Die Injektionsnadel 4a dient zum Injizieren der im Behälter 2 befindlichen Flüssigkeit 1.

Unter einem Haltemittel 4 zum Halten der Injektionsnadel 4a können im Zusammenhang mit der Erfindung alle erforderlichen Einheiten der Abgabevorrichtung dienen, die die Injektionsnadel 4a in ihrer Position gegenüber der Öffnung 3 des Behälters 2 halten. Dies kann insbesondere ein in der Öffnung 3 des Behälters 2 angeordnetes, und diesen im übrigen verschließendes, mittels einer Injektionsnadel 4adurchstechbares Septum 40 sein. Als Haltemittel 4 kann auch eine Kombination eines Außengewindes im Bereich der Öffnung mit einem fest mit der Injektionsnadel 4a verbundenen Halteteil 41 mit einem Innengewinde fungieren, durch die die Injektionsnadel 4a in ihrer Position gehalten wird.

In **Fig. 1** sind weiters zwei Messelektroden 9, 10 einer Messanordnung 5a dargestellt, mittels der ein Messwert M (**Fig. 17**) im Bereich des Behälters 2 bestimmt werden kann. Aufgrund dieses Messwertes M ist es im Prinzip möglich einen mit dem Behälter 2 oder der darin befindlichen Flüssigkeit 1 stehenden physikalischen oder chemischen Parameter P zu ermitteln. Bevorzugter Weise handelt es sich bei einem solchen Parameter P um den Füllstand der Flüssigkeit 1 im Behälter 2. Alternativ besteht auch die Möglichkeit, die Art der Flüssigkeit selbst, ihre Temperatur, ihren Druck oder ähnliche Werte aufgrund einer kapazitiven oder anderen sensorischen Messung im Bereich des Behälters 2 zu ermitteln.

Die in **Fig. 17** dargestellte Verschaltung sowie die einzelnen Komponenten sind allesamt auf der Abgabevorrichtung angeordnet. Ein Messsystem 5 ist an die beiden Messelektroden 9, 10 angeschlossen. Dem Ausgang des Systems liegt ein Messwert M an, aufgrund dessen der physikalische oder chemische Parameter P ermittelbar ist.

Um eine Verfälschung von Messergebnissen zu vermeiden, die aufgrund von physikalischer Berührung der Abgabevorrichtung von außen bewirkt wird, ist im vorliegenden Ausführungsbeispiel bevorzugt eine Abschirmung 11 vorgesehen, die außerhalb der beiden Messelektroden 9, 10 angeordnet ist und diese sowie den Behälter 2 umgibt.

Bei der vorliegenden Ausführungsform ist auf dem Behälter 2 ein Detektorelement 6 in Form einer Detektionselektrode 6 angeordnet. Wie aus **Fig. 1** ersichtlich handelt es sich bei der Detektionselektrode 6 um eine zwischen dem Behälter 2 und der Gehäusewand angeordnete metallische Platte oder Schleife, die als kapazitive Elektrode dient. Die Detektionselektrode 6 ist, wie in **Fig. 17** dargestellt, an eine Nadeldetektionsschaltung 8 in Form einer Kapazitätsmessschaltung 8 angeschlossen. Der zweite Anschluss der Kapazitätsmessschaltung 8 kann mit einer metallischen Struktur 7, insbesondere mit einer der der Messelektroden 9, 10, der Abschirmung 11, der Masse der Messystems 5 oder einer weiteren Detektionselektrode 7 (**Fig. 3** **bzw.** **Fig. 19**) verbunden sein. In der in **Fig. 17** dargestellten Ausführungsform ist der zweite Anschluss der Kapazitätsmessschaltung 8 mit der Abschirmung 11 elektrisch leitend verbunden. Ist eine Injektionsnadel 4a im Bereich der Haltemittel 4 im Bereich der Öffnung 3 vorhanden, wird zwischen der Detektionselektrode 6 und der Abschirmung 11 ein Detektionsergebnis in Form eines Kapazitätsmessergebnisses C ermittelt. Wird die Injektionsnadel 4a aus dem Haltemittel 4 entfernt, so wird mit der Kapazitätsmessschaltung 8 ein davon abweichendes Kapazitätsmessergebnis C ermittelt. Abhängig vom Kapazitätsmessergebnis C, das am Ausgang der Kapazitätsmessschaltung 8 zur Verfügung steht, ist es möglich, zu erkennen, ob sich die Injektionsnadel 4a im Haltemittel 4 im Bereich der Öffnung 3 befindet oder nicht. Dies kann vorteilhafterweise mittels eines Schwellenwertvergleichs ermittelt werden, wobei der Schwellenwert T gerade zwischen einer vorab bestimmten Kapazität bei Vorhandensein der Injektionsnadel 4a und einer davon abweichenden und vorab bestimmten Kapazität bei Fehlen der Injektionsnadel 4a gesetzt wird.

Sowohl der Messwert M als auch das Kapazitätsmessergebnis C sind einer Verarbeitungseinheit 13 zugeführt.

Die Verarbeitungseinheit 13, in einer konkreten Ausführungsform im Detail dargestellt in **Fig. 18****,** weist einen Schwellenwertvergleicher 18 auf. Das Kapazitätsmessergebnis C wird dem Schwellenwertvergleicher 18 zugeführt, der aufgrund des vorab vorgegebenen Schwellenwertes T eine Aussage darüber trifft, ob das Kapazitätsmessergebnis diesen Schwellenwert T überschreitet oder unterschreitet. Aufgrund des Vergleichs des Schwellenwerts T mit dem Kapazitätsmessergebnis C kann ermittelt, ob sich eine Injektionsnadel 4a in den Haltemitteln 4 befindet oder nicht. Der Vergleichswert V wird am Ausgang des Schwellenwertvergleichers 18 zur Verfügung gehalten.

In der in **Fig. 18** dargestellten Ausführungsform weist die Verarbeitungseinheit 13 weiters zwei Einheiten 14, 15 auf, denen jeweils der Messwert M zugeführt ist. Die Verarbeitungseinheit 13 weist eine erste Einheit 14 zur Bestimmung eines ersten Parameterwertes P1 und eine zweite Einheit 15 zur Bestimmung eines zweiten Parameterwertes P2 auf.
Je nachdem, ob die Injektionsnadel 4a in die Haltemittel 4 eingesteckt ist oder nicht, wird die Berechnung des Parameters P aufgrund des Messwerts M nach unterschiedlichen Berechnungsvorschriften vorgenommen. Es besteht eine erste Berechnungsvorschrift, die aufgrund des Messwerts M den Parameter P bei Vorhandensein der Injektionsnadel 4a in den Haltemitteln 4 liefert. Es besteht eine zweite Berechnungsvorschrift, die aufgrund des Messwerts M den Parameter P bei Fehlen der Injektionsnadel 4a in den Haltemitteln 4 liefert. Aufgrund des Messwerts M werden zwei Parameterwerte P1, P2 bestimmt, von denen der erste Parameterwert P1 nach der ersten Berechnungsvorschrift und der zweite Parameterwert P2 nach der zweiten Berechnungsvorschrift gebildet wird.

Die beiden Berechnungsvorschriften werden in den beiden Einheiten 14, 15 ausgeführt. Die erste Einheit 14 führt die erste Berechnungsvorschrift aus. An ihrem Ausgang liegt der erste Parameterwert P1 an, der den Parameter P korrekt wiedergibt, wenn sich die Injektionsnadel 4a in den Haltemitteln 4 befindet. Die zweite Einheit 15 führt die zweite Berechnungsvorschrift aus. An ihrem Ausgang liegt der zweite Parameterwert P2 an, der den Parameter P korrekt wiedergibt, wenn sich die Injektionsnadel 4a nicht in den Haltemitteln 4 befindet bzw. die Haltemittel 4 frei von einer Injektionsnadel 4a sind.

Die beiden Parameterwerte P1 und P2 sind einer Auswahleinheit 17 zugeführt. Zur Steuerung der Auswahleinheit 17 wird der vom Kapazitätsmesswert C abhängige Vergleichswert V des Schwellenwertvergleichers 18 herangezogen.
Ist eine Injektionsnadel 4a im Haltemittel 4 vorhanden wird am Ausgang der Vergleichseinheit 13 bzw. der Auswahleinheit 17 der erste Parameterwert P1 als Parameter P zur Verfügung gehalten, andernfalls wird der zweite Parameterwert P2 am Ausgang der Verarbeitungseinheit 13 als Parameter P zur Verfügung gehalten.

Besonders vorteilhaft können sowohl in der ersten Einheit 14 als auch in der zweiten Einheit 15 jeweils Kalibrierfunktionen F1, F2 abgespeichert werden, die die jeweilige erste und/oder zweite Berechnungsvorschrift als Zusammenhang zwischen dem Messwert M und dem mit dem Behälter oder der darin befindlichen Flüssigkeit in Zusammenhang stehenden physikalischen und chemischen Parameterwert P bei Vorhandensein oder Fehlen einer Injektionsnadel 4a im Haltemittel 4 beschreibt.

Die erste Einheit 14 wendet die erste Kalibrierfunktion F1 auf den bei ihr anliegenden Messwert M an. Das so erhaltene Ergebnis stellt sie an ihrem Ausgang als ersten Parameterwert P1 zur Verfügung. Die zweite Einheit 15 wendet die zweite Kalibrierfunktion F2 auf den bei ihr anliegenden Messwert M an. Das so erhaltene Ergebnis stellt sie an ihrem Ausgang als zweiten Parameterwert P2 zur Verfügung.

Alternativ zu der in **Fig. 18** dargestellten Ausführungsform der Verarbeitungseinheit 13 ist auch die in **Fig. 20** dargestellte Ausführungsform der Verarbeitungseinheit 13 möglich. In dieser Ausführungsform umfasst die Verarbeitungseinheit 13 nur eine Berechnungseinheit 140, sowie einen Speicher 150, der zwei unterschiedliche Kalibrierfunktionen F1 und F2 bereithält. Gemäß **Fig. 20** wird der Berechnungseinheit 140, in Abhängigkeit des Schwellwertvergleichsergebnisses V die jeweils passende Kalibrierfunktion zugeführt. Bei Zuführung der Kalibrierfunktion F1 wird von der Berechnungseinheit 140 ein Parameterwert berechnet und am Ausgang der Verarbeitungseinheit 13 zur Verfügung gehalten, der den Parameter P korrekt wiedergibt, wenn eine Injektionsnadel 4a im Haltemittel 4 vorhanden ist. Bei Zuführung der Kalibrierfunktion F2 wird von der Berechnungseinheit 140 ein Parameterwert berechnet und am Ausgang der Verarbeitungseinheit 13 zur Verfügung gehalten, der den Parameter P korrekt wiedergibt, wenn keine Injektionsnadel 4a im Haltemittel 4 vorhanden ist.

In den **Fig. 3 und 4** ist eine **zweite Ausführungsform** der Erfindung näher dargestellt. Bei dieser sind im Bereich der Öffnung 3 zwei einander an der Öffnung gegenüberliegende Detektionselektroden 6, 7 vorgesehen, aufgrund der Kapazität C zwischen diesen beiden Detektionselektroden 6, 7 kann das Vorhandensein bzw. Fehlen einer Injektionsnadel 4a im Haltemittel 4 im Bereich der Öffnung 3 festgestellt werden. Im übrigen entspricht diese Ausführungsform der ersten Ausführungsform der Erfindung. Die konkrete Anordnung der einzelnen zur Messung verwendeten Komponenten entspricht der in **Fig. 17** dargestellten Anordnung der ersten Ausführungsform der Erfindung mit dem in **Fig. 19** dargestellten Unterschied, dass beide Detektionselektroden 6, 7 an das Messsystem 5 angeschlossen sind.

Die in den **Fig. 5 und 6** dargestellte **dritte Ausführungsform** der Erfindung entspricht im wesentlichen der in den **Fig. 1 und 2** dargestellten ersten Ausführungsform der Erfindung. Wesentlicher Unterschied ist, dass die Detektionselektrode 6 im Außenbereich der Abgabevorrichtung vom Behälter 2 beabstandet angeordnet ist. Die Detektionselektrode 6 ist dabei in Form eines im Außenbereich des Behälters 2 angeordneten Elektrodenrings ausgebildet. Wie auch beim ersten Ausführungsbeispiel der Erfindung ist es möglich, an den zweiten Eingang der Kapazitätsmessschaltung 8, eine der Messelektroden 9, 10, die Abschirmung 11, die Masse des Messsystems 5 oder eine andere metallische Struktur anzuschließen.

Die in den **Fig. 7 und 8** dargestellte **vierte Ausführungsform** der Erfindung entspricht im wesentlichen der in den **Fig. 3 und 4** dargestellten zweiten Ausführungsform der Erfindung. Der wesentliche Unterschied zwischen der zweiten und vierten Ausführungsform der Erfindung liegt darin, dass die beiden Detektionselektroden 6, 7 im Außenbereich der Abgabevorrichtung vom Behälter 2 beabstandet angeordnet sind.

In den **Fig. 9 und 10** ist eine **fünfte Ausführungsform** der Erfindung näher dargestellt, die der ersten Ausführungsform der Erfindung bis auf die im Folgenden dargestellten Unterschiede entspricht. Die Abgabevorrichtung weist eine Kappe 16 auf. In dieser Kappe 16 kann gegebenenfalls eine Abschirmung 11 vorgesehen sein. Die Detektionselektrode 6 befindet sich im Stirnbereich der Kappe 16, die im vorliegenden Fall die Nadel 4a vollständig umgibt. Wie auch beim ersten und dritten Ausführungsbeispiel der Erfindung kann eine Kapazität zwischen der Detektionselektrode 6 und einer weiteren metallischen Struktur 7, 9, 10, 11, insbesondere eine der Messelektroden 9, 10 der Abschirmung 11 oder der Masse des Messsystems vorgenommen werden.

Die in den **Fig. 11 und 12** dargestellte **sechste Ausführungsform** der Erfindung entspricht der in **Fig. 3 und 4** dargestellten zweiten und vierten Ausführungsform der Erfindung, wobei anders als bei dieser die beiden Detektionselektroden im Inneren der Kappe 16 angeordnet sind. In dieser Kappe 16 kann gegebenenfalls eine Abschirmung 11 vorgesehen sein. Die Detektionselektroden 6, 7 befinden sich im Stirnbereich der Kappe 16, und liegen einander im Bereich der Injektionsnadel 4a gegenüber.

Bei einer **siebenten Ausführungsform** der Erfindung, dargestellt in den **Fig. 13 und 14** die im wesentlichen der **fünften Ausführungsform** der Erfindung entspricht, weist die Kappe 16 eine Ausnehmung 19 auf, durch die die Injektionsnadel 4a hindurchtritt. Die Detektionselektrode 6 ist ringförmig im inneren Stirnbereich der Kappe 16 ausgebildet und umgibt die von der Injektionsnadel 4a durchsetzte Ausnehmung 19.

In den **Fig. 15 und 16** ist eine **achte Ausführungsform** der Erfindung dargestellt, die im wesentlichen der sechsten Ausführungsform der Erfindung entspricht. Dabei ist in der Kappe 16 eine Ausnehmung 19 vorgesehen, durch die die Injektionsnadel 4a hindurchtreten kann. Die beiden Detektionselektroden 6, 7 befinden sich im Bereich der für die Injektionsnadel 4a vorgesehenen Ausnehmung 19.

**Fig. 21** zeigt eine alternative **neunte Ausführungsform** der Erfindung, die im wesentlichen der sechsten Ausführungsform der Erfindung entspricht. Die Detektion des Vorhandenseins oder Fehlens der Injektionsnadel 4a erfolgt in diesem Fall jedoch nicht über zwei kapazitive Elektroden sondern über zwei Elektroden 6, 7 die von einem gemeinsamen und an der Kappe 16 angeordneten Grundkörper 16a in Richtung der Injektionsnadel 4a abstehen. Ist eine elektrisch leitfähige Injektionsnadel 4a vorhanden, so schließt diese den Kontakt zwischen den beiden Elektroden 6, 7. Die Nadeldetektionsschaltung 8, die in diesem Fall die Leitfähigkeit zwischen den beiden Elektroden 6, 7 misst, detektiert bei Vorhandensein einer Injektionsnadel 4a eine Leitfähigkeit zwischen den Elektroden 6, 7, bei Fehlen der Injektionsnadel 4a wird hingegen keine Leitfähigkeit detektiert. Die Leitfähigkeit wird als Detektionsergebnis C am Ausgang der Nadeldetektionsschaltung 8 zur Verfügung gehalten.

**Fig. 22** zeigt eine **zehnte Ausführungsform** der Erfindung, die im wesentlichen der neunten Ausführungsform der Erfindung entspricht. Als Haltemittel 4 wird auch eine Kombination eines Außengewindes im Bereich der Öffnung mit einem fest mit der Injektionsnadel 4a verbundenen Halteteil 41 mit einem Innengewinde verwendet, durch die die Injektionsnadel 4a in ihrer Position gehalten wird. Im Außenbereich des das Innengewinde tragenden Halteteils ist eine leitfähige Schicht angeordnet.
Vom Innenbereich der Kappe 16 oder von einem mit der Kappe in Verbindung stehenden Grundkörper stehen zwei Messelektroden 6, 7 ab. Ist eine elektrisch leitfähige Injektionsnadel 4a vorhanden, so entsteht auch ein leitfähiger Kontakt zwischen den Messelektroden 6, 7 mit der auf dem Halteteil angeordneten leitfähigen Schicht. Die leitfähige Schicht schließt so den Kontakt zwischen den beiden Elektroden 6, 7.
Die Nadeldetektionsschaltung 8, die in diesem Fall die Leitfähigkeit zwischen den beiden Elektroden 6, 7 misst, detektiert bei Vorhandensein einer Injektionsnadel 4a eine Leitfähigkeit zwischen den Elektroden 6, 7, bei Fehlen der Injektionsnadel 4a wird hingegen keine Leitfähigkeit detektiert. Die Leitfähigkeit wird als Detektionsergebnis C am Ausgang der Nadeldetektionsschaltung 8 zur Verfügung gehalten.

**Fig. 23** zeigt eine **elfte Ausführungsform** der Erfindung, die im wesentlichen der zehnten Ausführungsform der Erfindung entspricht.
Als Haltemittel 4 wird auch eine Kombination eines Außengewindes im Bereich der Öffnung mit einem fest mit der Injektionsnadel 4a verbundenen Halteteil 41 mit einem Innengewinde verwendet, durch die die Injektionsnadel 4a in ihrer Position gehalten wird. Vom Innenbereich der Kappe 16 steht ein Taster 6a ab. Der das Innengewinde tragende Halteteil ragt in den Hubbereich des Tasters 6a und betätigt diesen.
Die Nadeldetektionsschaltung 8, ist dem Taster 6a nachgeschaltet und detektiert folglich, ob eine Injektionsnadel 4a vorhanden ist oder nicht. Die Leitfähigkeit wird als Detektionsergebnis C am Ausgang der Nadeldetektionsschaltung 8 zur Verfügung gehalten.

**Fig. 24** zeigt eine **zwölfte Ausführungsform** der Erfindung, die im wesentlichen der sechsten Ausführungsform der Erfindung entspricht. Anstelle der Detektionselektroden 6 und 7 ist eine Detektionsspule 6b vorhanden, die in einem Bereich angeordnet ist, in den - bei Vorhandensein - die Injektionsnadel 4a ragt, und deren beide Anschlüsse and die Nadeldetektionsschaltung angeschlossen sind Aufgrund der leitfähigen und/oder magnetischen Eigenschaften der Injektionsnadel 4a hat die Detektionsspule 6b eine bei Vorhandensein einer Injektionsnadel 4a eine andere Induktivität als bei Fehlen einer Injektionsnadel 4a.
Die Nadeldetektionsschaltung 8, die in diesem Fall die Induktivität der Detektionsspule 6b misst, hält diese Induktivität als Detektionsergebnis C an ihrem Ausgang zur Verfügung.

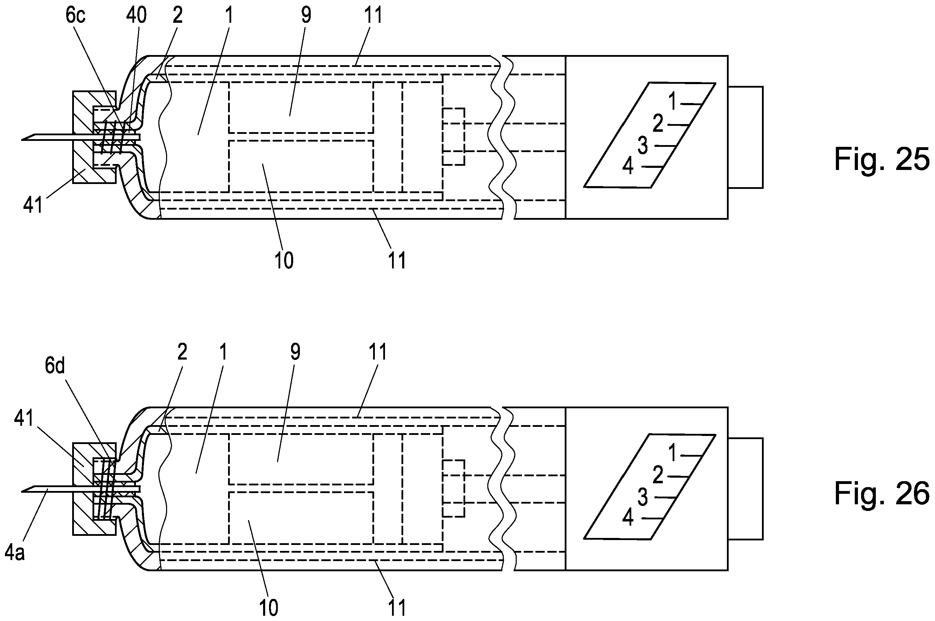

**Fig. 25** zeigt eine **dreizehnte Ausführungsform** der Erfindung, die im wesentlichen der zwölften Ausführungsform der Erfindung entspricht. Anders als in **Fig. 23** ist in **Fig. 24** die Detektionsspule 6c zwischen dem Behälter und der Gehäuseinnenwand der Abgabevorrichtung angeordnet. Wird eine Injektionsnadel 4a in die Öffnung 3 eingeführt bzw. durch das Septum hindurch geführt, ändert sich aufgrund der unterschiedlichen elektrischen und/oder magnetischen Eigenschaften der Injektionsnadel 4a die an der Detektionsspule 6c gemessene Induktivität als Detektionsergebnis C.

**Fig. 26** zeigt eine **vierzehnte Ausführungsform** der Erfindung, die im wesentlichen der dreizehnten Ausführungsform der Erfindung entspricht. Anders als in **Fig. 24** ist in **Fig. 25** die Detektionsspule 6d, beabstandet vom Behälter 2, zwischen der Abgabevorrichtung und dem das Innengewinde tragenden Teil des Haltemittels 4 angeordnet. Wird eine Injektionsnadel 4a in die Öffnung 3 eingeführt bzw. durch das Septum hindurch geführt, ändert sich aufgrund der unterschiedlichen elektrischen und/oder magnetischen Eigenschaften der Injektionsnadel 4a die an der Detektionsspule 6d gemessene Induktivität als Detektionsergebnis C.

## Patentansprüche

1. Abgabevorrichtung zur Abgabe von Flüssigkeiten (1), insbesondere von flüssigen Medikamenten an Personen, umfassend
- einen mit der Flüssigkeit (1) gefüllten Behälter (2), der an einem Ende eine Öffnung (3) zur Abgabe der Flüssigkeit (1) aufweist, wobei vorgesehen ist, dass im Bereich der Öffnung (3) Haltemittel (4) zum Halten einer elektrisch leitfähigen Injektionsnadel zum Injizieren der im Behälter (2) befindlichen Flüssigkeit (1) vorhanden sind,
- ein Messsystem (5) zur kapazitiven Bestimmung eines Messwerts (M), sowie
- eine Verarbeitungseinheit (13) zur Bestimmung zumindest eines in Zusammenhang mit dem Behälter (2) oder der darin befindlichen Flüssigkeit (1) stehenden physikalischen oder chemischen Parameterwert (P), insbesondere des Füllstands, aufgrund des Messwerts (M)
**dadurch gekennzeichnet,**
- **dass** zumindest ein Detektionselement (6) im Bereich der Öffnung (3), vorhanden ist,
- **dass** eine Nadeldetektionsschaltung (8) vorhanden ist, wobei das Detektionselement (6) an die Nadeldetektionsschaltung (8) angeschlossen ist,
- **dass** die Nadeldetektionsschaltung (8) zur Messung einer am Ausgang des Detektionselements (6) abgreifbaren elektrischen Kenngröße, insbesondere der Induktivität, Kapazität oder Leitfähigkeit, ausgebildet ist,
- **dass** eine Verarbeitungseinheit (13) vorhanden ist, und das am Ausgang der Nadeldetektionsschaltung (8) anliegende Messergebnis als Detektionsergebnis (C) der Verarbeitungseinheit (13) zugeführt ist, und
- **dass** die Verarbeitungseinheit (13) den Parameter (P) in Abhängigkeit des Messwertes (M) und des Detektionsergebnisses (C) berechnet und an ihrem Ausgang zur Verfügung hält.

2. Abgabevorrichtung nach Anspruch 1 **dadurch gekennzeichnet, dass** die Verarbeitungseinheit (13) das ihr zugeführte Detektionsergebnis (C) mit einem vorgegebenen Schwellenwert (T) vergleicht, und den am Ausgang zur Verfügung gehaltene Parameterwert (P) auf Basis des Messwertes (M) berechnet, wobei sie die zur Berechnung des Parameterwert (P) verwendete Berechnungsvorschrift abhängig vom Ergebnis des Vergleichs des Detektionsergebnisses (C) mit dem vorgegebenen Schwellenwert (T) ermittelt.

3. Abgabevorrichtung nach Anspruch 1 oder 2 **dadurch gekennzeichnet,**
- **dass** in der Verarbeitungseinheit (13) eine erste Kalibrierfunktion (F1) abgespeichert ist, die den Zusammenhang zwischen dem Messwert (M) und dem mit dem Behälter (2) oder der darin befindlichen Flüssigkeit (1) in Zusammenhang stehenden physikalischen oder chemischen Parameterwert (P) bei Vorhandensein einer Injektionsnadel im Haltemittel (4) angibt,
- **dass** in der Verarbeitungseinheit (13) eine zweite Kalibrierfunktion (F2) abgespeichert ist, die den Zusammenhang zwischen dem Messwert (M) und dem mit dem Behälter (2) oder der darin befindlichen Flüssigkeit (1) in Zusammenhang stehenden physikalischen oder chemischen Parameterwert (P) bei Fehlen einer Injektionsnadel im Haltemittel (4) angibt, und
- **dass** die Verarbeitungseinheit (13) die für die Berechnung des Parameters (P) verwendete Kalibrierfunktion in Abhängigkeit des Vergleichs des Detektionsergebnisses (C) mit dem vorgegebenen Schwellenwert (T) auswählt.

4. Abgabevorrichtung nach Anspruch 1 oder 2 **dadurch gekennzeichnet,**
- **dass** die Verarbeitungseinheit (13) eine erste Einheit (14) und eine zweite Einheit (15) umfasst,
- **dass** in der ersten Einheit (14) eine erste Kalibrierfunktion (F1) abgespeichert ist, die den Zusammenhang zwischen dem Messwert (M) und dem mit dem Behälter (2) oder der darin befindlichen Flüssigkeit (1) in Zusammenhang stehenden physikalischen oder chemischen Parameterwert (P) bei Vorhandensein einer Injektionsnadel im Haltemittel (4) angibt, wobei die erste Einheit (14) die erste Kalibrierfunktion (F1) auf den bei ihr anliegenden Messwert (M) anwendet und das so erhaltene Ergebnis an ihrem Ausgang als ersten Parameterwert (P1) zur Verfügung hält, und
- **dass** in der zweiten Einheit (15) eine zweite Kalibrierfunktion (F2) abgespeichert ist, die den Zusammenhang zwischen dem Messwert (M) und dem mit dem Behälter (2) oder der darin befindlichen Flüssigkeit (1) in Zusammenhang stehenden physikalischen oder chemischen Parameterwert (P) bei Fehlen einer Injektionsnadel im Haltemittel (4) angibt, wobei die zweite Einheit (15) die zweite Kalibrierfunktion (F2) auf den bei ihr anliegenden Messwert (M) anwendet und das so erhaltene Ergebnis an ihrem Ausgang als zweiten Parameterwert (P2) zur Verfügung hält.

5. Abgabevorrichtung nach einem der vorangegangenen Ansprüche **dadurch gekennzeichnet,**
- **dass** die Verarbeitungseinheit (13) eine erste Einheit (14) zur Bestimmung eines ersten Parameterwertes (P1) aufweist, die eine erste Berechnungsvorschrift, die aufgrund des Messwerts (M) den Parameterwert (P) bei Vorhandensein der Injektionsnadel (4a) in den Haltemitteln (4) liefert, auf den Messwert (M) anwendet,
- **dass** die Verarbeitungseinheit (13) eine zweite Einheit (15) zur Bestimmung eines zweiten Parameterwertes (P2) aufweist, die eine zweite Berechnungsvorschrift, die aufgrund des Messwerts (M) den Parameter (P) bei Fehlen der Injektionsnadel (4a) in den Haltemitteln (4) liefert, auf den Messwert (M) anwendet, und
- **dass** die Verarbeitungseinheit (13) das ihr zugeführte Detektionsergebnis (C) mit einem vorgegebenen Schwellenwert (T) vergleicht und abhängig von diesem Vergleich entweder den von der ersten Einheit (14) oder den von der zweiten Einheit (15) ermittelten ersten oder zweiten Parameter (P1, P2) als Parameter (P) an ihrem Ausgang zur Verfügung hält.

6. Abgabevorrichtung nach einem der vorangehenden Ansprüche, **dadurch gekennzeichnet, dass** an das Messsystem (5) zumindest ein Paar von im Außenbereich, insbesondere anliegend an der Wand des Behälters (2) angeordneten Messelektroden (9,10) zur Bestimmung der zwischen den Messelektroden (9,10) abgreifbaren Kapazität angeschlossen sind und dieser Kapazitätsmesswert als Messwert (M) der Verarbeitungseinheit (13) zugeführt ist,
- wobei insbesondere zumindest eine die Messelektroden (9,10) umgebende weitere Metallstruktur (11) vorhanden ist, und vorzugsweise zumindest eine der Metallstrukturen (11) als elektrische Abschirmung (12) ausgebildet ist.

7. Abgabevorrichtung nach einem der vorangehenden Ansprüche, umfassend eine aufsteckbare Kappe (16), **dadurch gekennzeichnet,**
- **dass** die aufsteckbare Kappe (16) den auf der Seite der Öffnung (3) befindlichen Bereich der Abgabevorrichtung zumindest teilweise und eine allenfalls von dem Haltemittel (4) gehaltene Injektionsnadel (4a) vollständig umgibt oder die eine Ausnehmung aufweist, durch die eine allenfalls von dem Haltemittel (4) gehaltene Injektionsnadel hindurch ragt,
- **dass** das Detektionselement (6) auf oder in der aufsteckbaren Kappe (16) im Bereich des Haltemittels (4) oder im Bereich der Injektionsnadel (4a) angeordnet ist, und
**dass** insbesondere das Messsystem (5) und die Nadeldetektionsschaltung (8) in der aufsteckbaren Kappe (16) integriert sind.

8. Abgabevorrichtung nach einem der vorangehenden Ansprüche, **gekennzeichnet dadurch,**
- **dass** das Detektionselement (6) als leitfähige im Bereich der Öffnung (3) angeordnete Detektionselektrode (6) ausgebildet ist,
- **dass** die Detektionselektrode (6) elektrisch an eine Nadeldetektionsschaltung (8) gekoppelt ist,
- **dass** die Nadeldetektionsschaltung (8) zur Messung der Kapazität zwischen der Detektionselektrode (6) und einer in oder auf der Abgabevorrichtung angeordneten weiteren metallischen Struktur (7) ausgebildet ist, wobei das am Ausgang der Nadeldetektionsschaltung (8) anliegende Kapazitätsmessergebnis als Detektionsergebnis (C) der Verarbeitungseinheit (13) zugeführt ist,
- **dass** die Verarbeitungseinheit (13) den Parameter (P) in Abhängigkeit des Messergebnisses (M) und des Detektionsergbnisses (C) berechnet und an ihrem Ausgang zur Verfügung hält.

9. Abgabevorrichtung nach einem der vorangehenden Ansprüche, **dadurch gekennzeichnet,**
- **dass** die metallische Struktur (7) als eine weitere Detektionselektrode (7) im Bereich der Öffnung (3), insbesondere im Bereich des Haltemittels (4) ausgebildet ist, oder
- **dass** als metallische Struktur (7) die Masse des Messsystems (5) fungiert, oder
- **dass** als metallische Struktur (7) zumindest eine der Messelektroden (9, 10) des Messsystems (5) fungiert, oder
- **dass** als metallische Struktur (7) eine weitere Metallstruktur (11) oder Abschirmung (12) fungiert,
oder dass als metallische Struktur (7) ein außen am Behälter (2) im Bereich der Öffnung (3) vorhandener Metallring fungiert

10. Abgabevorrichtung nach Anspruch 8 oder 9, **dadurch gekennzeichnet,**
- **dass** die Detektionselektrode (6) und gegebenenfalls die metallische Struktur (7), insbesondere in Form einer weiteren Detektionselektrode, in oder auf der aufsteckbaren Kappe (16) im Bereich des Haltemittels (4) oder im Bereich der Injektionsnadel (4a) angeordnet sind, und/oder
- **dass** die Detektionselektrode (6) sowie gegebenenfalls die metallische Struktur (7), insbesondere in Form einer weiteren Detektionselektrode, im Bereich der Haltemittel (4) für die Injektionsnadel an der Außenwand des Behälters (2) angeordnet sind, und/oder
- **dass** die Detektionselektrode (6) sowie gegebenenfalls die metallische Struktur (7), insbesondere in Form einer weiteren Detektionselektrode, im Bereich der Haltemittel (4) für die Injektionsnadel im Außenbereich der Abgabevorrichtung, beabstandet vom Behälter (2) angeordnet sind.

11. Abgabevorrichtung nach einem der Ansprüche 1 bis 7, **dadurch gekennzeichnet,**
- **dass** das Detektionselement (6) durch einen Schalter (6a) gebildet wird,
- **dass** die Nadeldetektionsschaltung (8) zur Messung der elektrischen Leitfähigkeit zwischen den Kontakten des Schalters (6a) ausgebildet ist,
- **dass** der Schalter (6a) durch das Vorhandensein einer Injektionsnadel (4a) mechanisch betätigt wird, und
- **dass** der Schalter (6a) an die Verarbeitungseinheit (13) elektrisch gekoppelt ist.

12. Abgabevorrichtung nach Anspruch 11, **dadurch gekennzeichnet,**
- **dass** aufgrund der Betätigung des Schalters (6a) dem Schwellwertvergleicher (18) der Verarbeitungseinheit (13) über den Schalter (6a) ein elektrisches Signal als Detektionsergebnis (C) zugeführt ist.

13. Abgabevorrichtung nach einem der Ansprüche 1 bis 7, **dadurch gekennzeichnet,**
- **dass** das Detektionselement (6) durch eine Leiteranordnung oder Spule (6b), gebildet wird, deren Induktivität bei Vorhandensein einer Injektionsnadel einen anderen Wert aufweist, als bei Fehlen der Injektionsnadel (4a),
- **dass** die Leiteranordnung oder Spule (6b, 6c, 6d) elektrisch an die als eine Induktivitätsmessschaltung (8) ausgebildete Nadeldetektionsschaltung (8) gekoppelt ist,
- **dass** die Nadeldetektionsschaltung (8) zur Messung der Induktivität der Leiteranordnung oder Spule (6b, 6c, 6d) vorhanden ist, wobei das am Ausgang der Nadeldetektionsschaltung (8) anliegende Detektionsergebnis (C) in Form eines Induktivitätsmessergebnisses der Verarbeitungseinheit (13) zugeführt ist,
- **dass** die Verarbeitungseinheit (13) den Parameter (P) in Abhängigkeit des Messergebnisses (M) und des Detektionsergebnisses (C) berechnet und an ihrem Ausgang zur Verfügung hält, wobei insbesondere
- die Leiteranordnung oder Spule (6b, 6c, 6d) im Bereich der Haltemittel (4) für die Injektionsnadel an der Außenwand des Behälters (2) angeordnet sind, oder
- **dass** die Leiteranordnung oder Spule (6b, 6c, 6d) im Bereich der Haltemittel (4) für die Injektionsnadel im Außenbereich der Abgabevorrichtung, beabstandet vom Behälter (2) angeordnet sind.

14. Abgabevorrichtung nach Anspruch 13, **dadurch gekennzeichnet,**
- **dass** die Spule (6b, 6c, 6d) derart angeordnet ist, dass bei Vorhandensein der Injektionsnadel (4a) diese von den Windungen der Spule zumindest teilweise umschlungen wird.

15. Abgabevorrichtung nach Anspruch 13, **dadurch gekennzeichnet,**
- **dass** die Leiteranordnung oder Spule (6b, 6c, 6d) in oder auf der aufsteckbaren Kappe (16) im Bereich des Haltemittels oder im Bereich der Injektionsnadel angeordnet sind, wobei insbesondere die Spule (6b, 6c, 6d) derart angeordnet ist, dass bei Vorhandensein der Injektionsnadel (4a) und auf die Abgabevorrichtung aufgesteckter Kappe (16), die Injektionsnadel (4a) von den Windungen der Spule zumindest teilweise umschlungen wird.

16. Abgabevorrichtung nach einem der Ansprüche 1 bis 7, **dadurch gekennzeichnet,**
- **dass** das Detektionselement (6) durch zwei Kontaktelemente (6, 7) gebildet wird, die bei Vorhandensein einer elektrisch leitfähigen Injektionsnadel (4a) mit dieser in elektrischem Kontakt stehen, wobei insbesondere über die elektrische Verbindung zwischen den Kontaktelementen (6, 7) und der elektrisch leitfähigen Nadel (4a) dem Schwellwertvergleicher (18) der Verarbeitungseinheit (13) ein elektrisches Signal als Detektionsergebnis (C) zugeführt wird.

17. Abgabevorrichtung nach einem der Ansprüche 1 bis 7, **dadurch gekennzeichnet,**
- **dass** auf einem mit der Injektionsnadel (4a) unlösbar verbundenem Halteteil (41) eine elektrisch leitfähige Beschichtung (42) vorhanden ist, und
- **dass** zwei Detektionselemente (6, 7) in Form von Kontaktelemente (6, 7) vorgesehen sind, die bei Vorhandensein einer Injektionsnadel (4a) mit der elektrisch leitfähigen Beschichtung in elektrischem Kontakt stehen, wobei insbesondere
- über die elektrische Verbindung zwischen den Kontaktelementen (6, 7) und der elektrisch leitfähigen Beschichtung dem Schwellwertvergleicher (18) der Verarbeitungseinheit (13) ein elektrisches Signal als Detektionsergebnis (C) zugeführt wird, und/oder
- die Kontaktelemente (6, 7) in oder auf der aufsteckbaren Kappe (16) im Bereich des Haltemittels (4) oder im Bereich der Injektionsnadel (4a) angeordnet sind.

18. Verfahren zur Bestimmung eines in Zusammenhang mit einem Behälter (2) oder einer in dem Behälter (2) befindlichen Flüssigkeit (1) stehenden physikalischen oder chemischen Parameters (P), insbesondere des Füllstands der Flüssigkeit im Behälter (2),
- wobei der Behälter (2) an einem Ende eine Öffnung (3) zur Abgabe der Flüssigkeit (1) aufweist, und wobei im Bereich der Öffnung (3) Haltemittel (4) zum Halten einer elektrisch leitfähigen Injektionsnadel (4a) zum Injizieren der im Behälter (2) befindlichen Flüssigkeit (1) vorhanden sind, und
- wobei ein Messwert (M) im Bereich des Behälters (2) ermittelt wird,
**dadurch gekennzeichnet,**
- **dass** mittels eines im Bereich der Öffnung (3) befindlichen Detektionselementes (6) ein vom Vorhandensein einer Injektionsnadel (4a) abhängiges Detektionsergebnis (C), ermittelt wird durch Ermittlung der Kapazität zwischen einer im Bereich der Öffnung (3) befindlichen metallischen Detektionselektrode (6) und einer in oder auf der Abgabevorrichtung angeordneten weiteren metallischen Struktur (7),
- **dass** das Detektionsergebnis (C) mit einem Schwellenwert (T) verglichen wird und abhängig vom Schwellenwertvergleich das Vorhandensein oder das Fehlen einer Injektionsnadel (4a) im Haltemittel festgestellt wird, wobei
- bei Detektion des Vorhandenseins einer Injektionsnadel ein erster Parameter (P1) durch Anwendung einer ersten Berechnungsvorschrift, die aufgrund des Messwerts (M) den Parameterwert (P) bei Vorhandensein der Injektionsnadel (4a) in den Haltemitteln (4) liefert, auf den Messwert (M) angewendet wird, und der so ermittelte erste Parameterwert (P1) als Parameterwert (P) zur Verfügung gehalten wird, und
- bei Detektion des Fehlens einer Injektionsnadel ein zweiter Parameterwert (P2) durch Anwendung einer zweiten Berechnungsvorschrift, die aufgrund des Messwerts (M) den Parameterwert (P) bei Fehlen der Injektionsnadel (4a) in den Haltemitteln (4) liefert, auf den Messwert (M) angewendet wird, und der so ermittelte zweite Parameter (P2) als Parameterwert (P) zur Verfügung gehalten wird.

19. Verfahren nach Anspruch 18, **dadurch gekennzeichnet,**
- **dass** eine erste Kalibrierfunktion (F1) vorgegeben wird, die den Zusammenhang zwischen dem Messwert (M) und dem mit dem Behälter (2) oder der darin befindlichen Flüssigkeit (1) in Zusammenhang stehenden physikalischen oder chemischen Parameterwert (P) bei Vorhandensein einer Injektionsnadel im Haltemittel (4) angibt,
- **dass** eine zweite Kalibrierfunktion (F2) vorgegeben wird, die den Zusammenhang zwischen dem Messwert (M) und dem mit dem Behälter (2) oder der darin befindlichen Flüssigkeit (1) in Zusammenhang stehenden physikalischen oder chemischen Parameterwert (P) bei Fehlen einer Injektionsnadel im Haltemittel (4) angibt, und
- **dass** bei Detektion des Vorhandenseins der einer Injektionsnadel (4a) die erste Kalibrierfunktion (F1) auf den Messwert (M) angewendet wird und das so erhaltene Ergebnis als Parameter (P) zur Verfügung gehalten wird, und
- **dass** bei Detektion des Fehlens der einer Injektionsnadel (4a) die zweite Kalibrierfunktion (F2) auf den Messwert (M) angewendet wird und das so erhaltene Ergebnis als Parameterwert (P) zur Verfügung gehalten wird.

20. Verfahren nach einem der Ansprüche 18 oder 19, **dadurch gekennzeichnet, dass** als Messwert (M) die zwischen zwei im Außenbereich, insbesondere anliegend an der Wand des Behälters (2), angeordneten Messelektroden (9,10), abgreifbare Kapazität herangezogen wird.

## Claims

1. A dispensing device for dispensing liquids (1), in particular liquid drugs to persons, comprising
- a container (2) filled with the liquid (1), which at one end has an opening (3) for dispensing the liquid (1), wherein in the region of the opening (3) there are retaining means (4) for holding an electrically conductive injection needle for injecting the liquid (1) contained in the container (2),
- a measuring system (5) for capacitive determination of a measurement value (M), and
- a processing unit (13) for determining at least one physical or chemical parameter value (P) associated with the container (2) or the liquid (1) contained therein, in particular the filling level, by means of the measurement value (M),
**characterised in**
- **that** there is at least one detection element (6) in the region of the opening (3),
- **that** there is a needle detection circuit (8), wherein the detection element (6) is connected to the needle detection circuit (8),
- **that** the needle detection circuit (8) is designed to measure an electrical parameter, in particular the inductance, capacitance or conductivity, which can be tapped at the outlet of the detection element (6),
- **that** there is a processing unit (13), and the measurement result at the outlet of the needle detection circuit (8) is supplied as a detection result (C) to the processing unit (13), and
- **that** the processing unit (13) calculates the parameter (P) according to the measurement value (M) and the detection result (C) and provides said parameter at the outlet thereof.

2. The dispensing device according to claim 1, **characterised in that** the processing unit (13) compares the detection result (C) supplied thereto with a predetermined threshold value (T), and calculates the parameter value (P) provided at the outlet on the basis of the measurement value (M), wherein said processing unit determines the calculation rule used to calculate the parameter (P) according to the result of the comparison of the detection result (C) with the predetermined threshold value (T).

3. The dispensing device according to claim 1 or 2, **characterised in**
- **that** in the processing unit (13) a first calibration function (F1) is stored, which states the relationship between the measurement value (M) and the physical or chemical parameter value (P) associated with the container (2) or the liquid (1) contained therein when an injection needle is present in the retaining means (4),
- **that** in the processing unit (13) a second calibration function (F2) is stored, which states the relationship between the measurement value (M) and the physical or chemical parameter value (P) associated with the container (2) or the liquid (1) contained therein when an injection needle in the retaining means (4) is absent, and
- **that** the processing unit (13) selects the calibration function used for calculating the parameter (P) according to the comparison of the detection result (C) with the predetermined threshold value (T).

4. The dispensing device according to claim 1 or 2, **characterised in**
- **that** the processing unit (13) comprises a first unit (14) and a second unit (15),
- **that** in the first unit (14) a first calibration function (F1) is stored, which states the relationship between the measurement value (M) and the physical or chemical parameter value (P) associated with the container (2) or the liquid (1) contained therein when an injection needle is present in the retaining means (4), wherein the first unit (14) applies the first calibration function (F1) to the measurement value (M) present therefor and provides the result obtained in this manner at the outlet thereof as the first parameter value (P1), and
- **that** in the second unit (15) a second calibration function (F2) is stored, which states the relationship between the measurement value (M) and the physical or chemical parameter value (P) associated with the container (2) or the liquid (1) contained therein when an injection needle in the retaining means (4) is absent, wherein the second unit (15) applies the second calibration function (F2) to the measurement value (M) present therefor and provides the result obtained in this manner at the outlet thereof as the second parameter value (P2).

5. The dispensing device according to one of the preceding claims, **characterised in**
- **that** the processing unit (13) has a first unit (14) for determining a first parameter value (P1), which applies a first calculation rule, which, on the basis of the measurement value (M), applies the parameter value (P), when the injection needle (4a) is present in the retaining means (4), to the measurement (M),
- **that** the processing unit (13) has a second unit (15) for determining a second parameter value (P2), which applies a second calculation rule, which on the basis of the measurement value (M) applies the parameter value (P), when the injection needle (4a) in the retaining means (4) is absent, to the measurement value (M), and
- **that** the processing unit (13) compares the detection result (C) supplied thereto with a predetermined threshold value (T) and according to said comparison provides either the first or second parameter (P1, P2) determined by the first unit (14) or the second unit (15) as the parameter (P) at the outlet thereof.

6. The dispensing device according to one of the preceding claims, **characterised in that** on the measuring system (5) at least one pair of measuring electrodes (9, 10) arranged in the outer region, in particular abutting the wall of the container (2), is connected for determining the capacitance which can be tapped between the measuring electrodes (9, 10), and said capacitance measurement is supplied as a measurement value (M) to the processing unit (13),
- wherein there is in particular at least one further metal structure (11) surrounding the measuring electrodes (9, 10), and preferably at least one of the metal structures (11) is designed as electrical shielding (12).

7. The dispensing device according to one of the preceding claims, comprising an attachable cap (16), **characterised in**
- **that** the attachable cap (16) at least partially surrounds the region of the dispensing device disposed on the side of the opening (3) and completely surrounds an injection needle (4a) held as needed by the retaining means (4) or has a recess, through which an injection needle held as needed by the retaining means (4) protrudes,
- **that** the detection element (6) is arranged on or in the attachable cap (16) in the region of the retaining means (4) or in the region of the injection needle (4a), and
**that** in particular the measuring system (5) and the needle detection circuit (8) are integrated in the attachable cap (16).

8. The dispensing device according to one of the preceding claims, **characterised in**
- **that** the detection element (6) is designed as a conductive detection electrode (6) arranged in the region of the opening (3),
- **that** the detection electrode (6) is coupled electrically to a needle detection circuit (8),
- **that** the needle detection circuit (8) is designed for measuring the capacitance between the detection electrode (6) and a further metallic structure (7) arranged in or on the dispensing device, wherein the capacitance measurement result at the outlet of the needle detection circuit (8) is supplied as a detection result (C) to the processing unit (13),
- **that** the processing unit (13) calculates the parameter (P) according to the measurement result (M) and the detection result (C) and provides same at the outlet thereof.

9. The dispensing device according to one of the preceding claims, **characterised in**
- **that** the metallic structure (7) is designed as a further detection electrode (7) in the region of the opening (3), in particular in the region of the retaining means (4), or
- **that** the mass of the measuring system (5) acts as the metallic structure (7), or
- **that** at least one of the measuring electrodes (9, 10) of the measuring system (5) acts as the metallic structure (7), or
- **that** a further metallic structure (11) or shielding (12) acts as the metallic structure (7),
or that a metal ring on the outside of the container (2) in the region of the opening (3) acts as the metallic structure (7).

10. The dispensing device according to claim 8 or 9, **characterised in**
- **that** the detection electrode (6) and, where applicable, the metallic structure (7), in particular in the form of a further detection electrode, are arranged in or on the attachable cap (16) in the region of the retaining means (4) or in the region of the injection needle (4a), and/or
- **that** the detection electrode (6) and, where applicable, the metallic structure (7), in particular in the form of a further detection electrode, are arranged in the region of the retaining means (4) for the injection needle on the outer wall of the container (2), and/or
- **that** the detection electrode (6) and, where applicable, the metallic structure (7), in particular in the form of a further detection electrode, are arranged in the region of the retaining means (4) for the injection needle in the outer region of the dispensing device, at a distance from the container (2).

11. The dispensing device according to one of claims 1 to 7, **characterised in**
- **that** the detection element (6) is formed by a switch (6a),
- **that** the needle detection circuit (8) is designed for measuring the electrical conductivity between the contacts of the switch (6a),
- **that** the switch (6a) is mechanically actuated by the presence of an injection needle (4a), and
- **that** the switch (6a) is electrically coupled to the processing unit (13).

12. The dispensing device according to claim 11, **characterised in**
- **that** on the basis of actuation of the switch (6a) an electrical signal as a detection result (C) is supplied to the threshold value comparator (18) of the processing unit (13) by the switch (6a).

13. The dispensing device according to one of claims 1 to 7, **characterised in**
- **that** the detection element (6) is formed by a conductor arrangement or coil (6b), the inductance of which has a different value when an injection needle is present than when the injection needle (4a) is absent,
- **that** the conductor arrangement or coil (6b, 6c, 6d) is coupled electrically to the needle detection circuit (8) formed as an inductance measurement circuit (8),
- **that** the needle detection circuit (8) is provided for measuring the inductance of the conductor arrangement or coil (6b, 6c, 6d), wherein the detection result (C) at the outlet of the needle detection circuit (8) is supplied to the processing unit (13) in the form of an inductance measurement result,
- **that** the processing unit (13) calculates the parameter (P) according to the measurement result (M) and the detection result (C) and provides same at the outlet thereof, wherein in particular
- the conductor arrangement or coil (6b, 6c, 6d) is arranged in the region of the retaining means (4) for the injection needle on the outer wall of the container (2), or
- the conductor arrangement or coil (6b, 6c, 6d) is arranged in the region of the retaining means (4) for the injection needle in the outer region of the dispensing device, at a distance from the container (2).

14. The dispensing device according to claim 13, **characterised in**
- **that** the coil (6b, 6c, 6d) is arranged such that when the injection needle (4a) is present, same is at least partially entwined by the windings of the coil.

15. The dispensing device according to claim 13, **characterised in**
- **that** the conductor arrangement or coil (6b, 6c, 6d) is arranged in or on the attachable cap (16) in the region of the retaining means or in the region of the injection needle, wherein in particular the coil (6b, 6c, 6d) is arranged such that when the injection needle (4a) is present and the cap (16) is attached to the dispensing device, the injection needle (4a) is at least partially entwined by the windings of the coil.

16. The dispensing device according to one of claims 1 to 7, **characterised in**
- **that** the detection element (6) is formed by two contact elements (6, 7) which, when an electrically conductive injection needle (4a) is present, are in electrical contact with said injection needle, wherein in particular by means of the electrical connection between the contact elements (6, 7) and the electrically conductive needle (4a) an electrical signal (C) is supplied as a detection result to the threshold value comparator (18) of the processing unit (13).

17. The dispensing device according to one of claims 1 to 7, **characterised in**
- **that** there is an electrically conductive coating (42) on a retaining part (41) non-detachably connected to the injection needle (4a), and
- **that** there are two detection elements (6, 7) in the form of contact elements (6, 7), which are in electrical contact with the electrically conductive coating when an injection needle (4a) is present, wherein in particular
- by means of the electrical connection between the contact elements (6, 7) and the electrically conductive coating an electrical signal is supplied as a detection result (C) to the threshold value comparator (18) of the processing unit (13), and/or
- the contact elements (6, 7) are arranged in or on the attachable cap (16) in the region of the retaining means (4) or in the region of the injection needle (4a).

18. A method for determining a physical or chemical parameter (P) associated with the container (2) or the liquid (1) contained in the container (2), in particular the filling level of the liquid in the container (2),
- wherein the container (2) at one end has an opening (3) for dispensing the liquid (1), and wherein in the region of the opening (3) there are retaining means (4) for holding an electrically conductive injection needle (4a) for injecting the liquid (1) contained in the container (2), and
- wherein a measurement value (M) is determined in the region of the container (2),
**characterised in**
- **that** by means of a detection element (6) disposed in the region of the opening (3) a detection result (C) dependent on the presence of an injection needle (4a) is determined by determining the capacitance between a metallic detection electrode (6) disposed in the region of the opening (3) and a further metallic structure (7) arranged in or on the dispensing device,
- **that** the detection result (C) is compared with a threshold value (T) and, according to the threshold value comparison, the presence or absence of an injection needle (4a) in the retaining means is identified, wherein
- when the presence of an injection needle is detected, a first parameter value (P1) is applied to the measurement value (M) by applying a first calculation rule, which on the basis of the measurement value (M) provides the parameter value (P) when the injection needle (4a) is present in the retaining means (4), and the first parameter value (P1) determined in this manner is provided as a parameter value (P), and
- when the absence of an injection needle is detected, a second parameter value (P2) is applied to the measurement value (M) by applying a second calculation rule, which on the basis of the measurement (M) provides the parameter value (P) in the absence of the injection needle (4a) in the retaining means (4), and the second parameter value (P2) determined in this manner is provided as a parameter value (P).

19. The method according to claim 18, **characterised in**
- **that** a first calibration function (F1) is predetermined, which states the relationship between the measurement value (M) and the physical or chemical parameter value (P) associated with the container (2) or the liquid (1) contained therein when an injection needle is present in the retaining means (4),
- **that** a second calibration function (F2) is predetermined, which states the relationship between the measurement value (M) and the physical or chemical parameter value (P) associated with the container (2) or the liquid (1) contained therein when an injection needle in the retaining means (4) is absent, and
- **that** when the presence of an injection needle (4a) is detected, the first calibration function (F1) is applied to the measurement value (M) and the result obtained in this manner is provided as the parameter (P), and
- **that** when the absence of an injection needle (4a) is detected, the second calibration function (F2) is applied to the measurement value (M) and the result thus obtained is provided as the parameter value (P).

20. The method according to one of claims 18 or 19, **characterised in that** the capacitance which can be tapped between two measuring electrodes (9, 10) arranged in the outer region, in particular abutting the wall of the container (2), is used as a measurement value.

## Revendications

1. Dispositif d'administration servant à administrer des liquides (1), notamment des médicaments liquides, à des personnes, comprenant
- un récipient (2) rempli du liquide (1), qui présente à une extrémité une ouverture (3) pour l'administration du liquide (1), un moyen de retenue (4) étant prévu dans la zone de l'ouverture (3) pour retenir une aiguille d'injection électroconductrice, destinée à injecter le liquide (1) se trouvant dans le récipient (2),
- un système de mesure (5) destiné à la détermination capacitive d'une valeur de mesure (M), ainsi que
- une unité de traitement (13) destinée à la détermination d'au moins une valeur de paramètre physique ou chimique (P), en liaison avec le récipient (2) ou le liquide (1) se trouvant dans ce dernier, en particulier du niveau de remplissage, sur la base de la valeur de mesure (M)
**caractérisé en ce**
- **qu'**au moins un élément de détection (6) est présent dans la zone de l'ouverture (3),
- en ce qu'un circuit (8) de détection d'aiguille est présent, l'élément de détection (6) étant connecté au circuit (8) de détection d'aiguille,
- en ce que le circuit (8) de détection d'aiguille est conçu pour la mesure d'une variable électrique pouvant être prélevée en sortie de l'élément de détection (6), en particulier l'inductance, la capacité ou la conductivité,
- en ce qu'une unité de traitement (13) est présente et le résultat de mesure, appliqué en sortie du circuit (8) de détection d'aiguille, est envoyé en tant que résultat de détection (C) à l'unité de traitement (13) et
- en ce que l'unité de traitement (13) calcule le paramètre (P) en fonction de la valeur de mesure (M) et du résultat de détection (C) et le met à disposition en sortie.

2. Dispositif d'administration selon la revendication 1, **caractérisé en ce que** l'unité de traitement (13) compare à une valeur seuil prédéfinie (T) le résultat de détection (C) qui lui a été envoyé et calcule sur la base de la valeur de mesure (M) la valeur du paramètre (P), mise à disposition en sortie, l'unité de traitement déterminant la règle de calcul utilisée pour le calcul de la valeur du paramètre (P) en fonction du résultat de la comparaison du résultat de détection (C) et de la valeur seuil prédéfinie.

3. Dispositif d'administration selon la revendication 1 ou 2, **caractérisé en ce**
- **que** dans l'unité de traitement (13) est stockée une première fonction d'étalonnage (F1), qui indique la relation entre la valeur de mesure (M) et la valeur du paramètre physique ou chimique (P) en liaison avec le récipient (2) ou le liquide (1) se trouvant dans ce dernier, en présence d'une aiguille d'injection dans le moyen de retenue (4),
- en ce que dans l'unité de traitement (13) est stockée une seconde fonction d'étalonnage (F2), qui indique la relation entre la valeur de mesure (M) et la valeur du paramètre physique ou chimique (P) en liaison avec le récipient (2) ou le liquide (1) se trouvant dans ce dernier, en l'absence d'aiguille d'injection dans le moyen de retenue (4) et
- en ce que l'unité de traitement (13) choisit la fonction d'étalonnage utilisée pour le calcul du paramètre (P) en fonction de la comparaison du résultat de détection (C) et de la valeur seuil prédéfinie (T).

4. Dispositif d'administration selon la revendication 1 ou 2, **caractérisé en ce**
- **que** l'unité de traitement (13) comprend une première unité (14) et une seconde unité (15),
- en ce que dans la première unité (14) est stockée une première fonction d'étalonnage (F1), qui indique la relation entre la valeur de mesure (M) et la valeur du paramètre physique ou chimique (P) en liaison avec le récipient (2) ou le liquide (1) se trouvant dans ce dernier, en présence d'une aiguille d'injection dans le moyen de retenue (4), la première unité (14) utilisant la première fonction d'étalonnage (F1) sur la valeur de mesure (M) qui lui est appliquée et mettant à disposition en sortie le résultat ainsi obtenu, en tant que première valeur du paramètre (P1) et
- en ce que dans la seconde unité (15) est stockée une seconde fonction d'étalonnage (F2), qui indique la relation entre la valeur de mesure (M) et la valeur du paramètre physique ou chimique (P) en liaison avec le récipient (2) ou le liquide (1) se trouvant dans ce dernier, en l'absence d'aiguille d'injection dans le moyen de retenue (4), la seconde unité (15) utilisant la seconde fonction d'étalonnage (F2) sur la valeur de mesure (M) qui lui est appliquée et mettant à disposition en sortie le résultat ainsi obtenu en tant que seconde valeur du paramètre (P2).

5. Dispositif d'administration selon l'une des revendications précédentes, **caractérisé en ce**
- **que** l'unité de traitement (13) présente une première unité (14) destinée à la détermination d'une première valeur du paramètre (P1), qui utilise sur la valeur de mesure (M) une première règle de calcul, qui sur la base de la valeur de mesure (M) fournit la valeur du paramètre (P) en présence de l'aiguille d'injection (4a) dans le moyen de retenue (4),
- en ce que l'unité de traitement (13) présente une seconde unité (15) destinée à la détermination d'une seconde valeur du paramètre (P2), qui utilise sur la valeur de mesure (M) une seconde règle de calcul, qui sur la base de la valeur de mesure (M) fournit le paramètre (P) en l'absence d'aiguille d'injection (4a) dans le moyen de retenue (4) et
- en ce que l'unité de traitement (13) compare le résultat de détection (C) qui lui est envoyé à une valeur seuil prédéfinie (T) et, en fonction de cette comparaison, met à disposition en sortie en tant que paramètre (P) le premier ou le second paramètre (P1, P2) déterminé par la première unité (14) ou par la seconde unité (15).

6. Dispositif d'administration selon l'une des revendications précédentes, **caractérisé en ce qu'**au système de mesure (5) sont connectées au moins une paire d'électrodes de mesure (9, 10), disposées dans la zone extérieure, en particulier s'appuyant contre la paroi du récipient (2), destinées à la détermination de la capacité pouvant être prélevée entre les électrodes de mesure (9, 10) et cette valeur de mesure de capacité étant envoyée en tant que valeur de mesure (M) à l'unité de traitement (13),
- en particulier au moins une structure métallique supplémentaire (11) étant prévue, qui entoure les électrodes de mesure (9, 10) et de préférence au moins l'une des structures métalliques (11) étant conçue comme un blindage électrique (12).

7. Dispositif d'administration selon l'une des revendications précédentes, comprenant un capuchon emboîtable (16), **caractérisé en ce**
- **que** le capuchon emboîtable (16) entoure au moins partiellement la zone du dispositif d'administration se trouvant du côté de l'ouverture (3) et entoure complètement une aiguille d'injection (4a) éventuellement retenue par le moyen de retenue (4) ou présente un évidement, par lequel dépasse une aiguille d'injection éventuellement retenue par le moyen de retenue (4),
- en ce que l'élément de détection (6) est disposé sur ou dans le capuchon emboîtable (16) dans la zone du moyen de retenue (4) ou dans la zone de l'aiguille d'injection (4a) et
en ce qu'en particulier le système de mesure (5) et le circuit (8) de détection d'aiguille sont intégrés dans le capuchon emboîtable (16).

8. Dispositif d'administration selon l'une des revendications précédentes, **caractérisé en ce**
- **que** l'élément de détection (6) est conçu comme une électrode de détection (6) conductrice, disposée dans la zone de l'ouverture (3),
- en ce que l'électrode de détection (6) est électriquement couplée à un circuit (8) de détection d'aiguille,
- en ce que le circuit (8) de détection d'aiguille est conçu pour la mesure de la capacité entre l'électrode de détection (6) et une structure métallique supplémentaire (7) disposée dans ou sur le dispositif d'administration, le résultat de la mesure de capacité, appliqué en sortie du circuit (8) de détection d'aiguille, étant envoyé en tant que résultat de détection (C) à l'unité de traitement (13),
- en ce que l'unité de traitement (13) calcule le paramètre (P) en fonction du résultat de mesure (M) et du résultat de détection (C) et le met à disposition en sortie.

9. Dispositif d'administration selon l'une des revendications précédentes, **caractérisé en ce**
- **que** la structure métallique (7) est conçue comme une électrode de détection supplémentaire (7) dans la zone de l'ouverture (3), en particulier dans la zone du moyen de retenue (4) ou
- en ce que c'est la masse du système de mesure (5) qui joue le rôle de structure métallique (7) ou
- en ce que c'est au moins l'une des électrodes de mesure (9, 10) du système de mesure (5) qui joue le rôle de structure métallique (7) ou
- en ce que c'est une structure métallique supplémentaire (11) ou un blindage (12) qui joue le rôle de structure métallique (7)
ou en ce que c'est un anneau métallique, présent à l'extérieur du récipient (2) dans la zone de l'ouverture (3), qui joue le rôle de structure métallique (7).

10. Dispositif d'administration selon la revendication 8 ou 9, **caractérisé en ce**
- **que** l'électrode de détection (6) et éventuellement la structure métallique (7), en particulier sous forme d'une électrode de détection supplémentaire, sont disposées dans ou sur le capuchon emboîtable (16) dans la zone du moyen de retenue (4) ou dans la zone de l'aiguille d'injection (4a) et/ou
- en ce que l'électrode de détection (6), ainsi qu'éventuellement la structure métallique (7), en particulier sous forme d'une électrode de détection supplémentaire, sont disposées dans la zone du moyen de retenue (4) de l'aiguille d'injection contre la paroi extérieure du récipient (2) et/ou
- en ce que l'électrode de détection (6), ainsi qu'éventuellement la structure métallique (7), en particulier sous forme d'une électrode de détection supplémentaire, sont disposées dans la zone du moyen de retenue (4) de l'aiguille d'injection dans la zone extérieure du dispositif d'administration, à distance du récipient (2).

11. Dispositif d'administration selon l'une des revendications 1 à 7, **caractérisé en ce**
- **que** l'élément de détection (6) est formé d'un commutateur (6a),
- en ce que le circuit (8) de détection d'aiguille est conçu pour la mesure de la conductivité électrique entre les contacts du commutateur (6a),
- en ce que le commutateur (6a) est mécaniquement actionné par la présence d'une aiguille d'injection (4a) et
- en ce que le commutateur (6a) est électriquement couplé à l'unité de traitement (13).

12. Dispositif d'administration selon la revendication 11, **caractérisé en ce**
- **que** sur la base de l'actionnement du commutateur (6a), un signal électrique est envoyé en tant que résultat de détection (C) au comparateur de valeur seuil (18) de l'unité de traitement (13) par l'intermédiaire du commutateur (6a).

13. Dispositif d'administration selon l'une des revendications 1 à 7, **caractérisé en ce**
- **que** l'élément de détection (6) est formé d'un ensemble de conducteurs ou d'une bobine (6b), dont l'inductance, en présence d'une aiguille d'injection, présente une valeur différente qu'en l'absence de l'aiguille d'injection (4a),
- en ce que l'ensemble de conducteurs ou la bobine (6b, 6c, 6d) est couplé(e) électriquement au circuit (8) de détection d'aiguille conçu comme un circuit de mesure de l'inductance (8),
- en ce que le circuit (8) de détection d'aiguille est présent pour la mesure de l'inductance de l'ensemble de conducteurs ou de la bobine (6b, 6c, 6d), le résultat de détection (C), appliqué en sortie du circuit (8) de détection d'aiguille, étant envoyé sous forme d'un résultat de mesure de l'inductance à l'unité de traitement (13),
- en ce que l'unité de traitement (13) calcule le paramètre (P) en fonction du résultat de mesure (M) et du résultat de détection (C) et le met à disposition en sortie,
- en particulier l'ensemble de conducteurs ou la bobine (6b, 6c, 6d) étant disposé(e) dans la zone du moyen de retenue (4) de l'aiguille d'injection contre la paroi extérieure du récipient (2) ou
- en ce que l'ensemble de conducteurs ou la bobine (6b, 6c, 6d) est disposé(e) dans la zone du moyen de retenue (4) de l'aiguille d'injection dans la zone extérieure du dispositif d'administration, à distance du récipient (2).

14. Dispositif d'administration selon la revendication 13, **caractérisé en ce**
- **que** la bobine (6b, 6c, 6d) est disposée de telle sorte qu'en présence de l'aiguille d'injection (4a), les spires de la bobine s'enroulent au moins partiellement autour de cette dernière.

15. Dispositif d'administration selon la revendication 13, **caractérisé en ce**
- **que** l'ensemble de conducteurs ou la bobine (6b, 6c, 6d) est disposé(e) dans ou sur le capuchon emboîtable (16) dans la zone du moyen de retenue ou dans la zone de l'aiguille d'injection, en particulier la bobine (6b, 6c, 6d) étant disposée de telle sorte qu'en présence de l'aiguille d'injection (4a) et sur le capuchon (16) emboîté sur le dispositif d'administration, les spires de la bobine s'enroulent au moins partiellement autour de l'aiguille d'injection (4a).

16. Dispositif d'administration selon l'une des revendications 1 à 7, **caractérisé en ce**
- **que** l'élément de détection (6) est formé par deux éléments de contact (6, 7), qui en présence d'une aiguille d'injection électroconductrice (4a) sont en contact électrique avec cette dernière, en particulier un signal électrique étant envoyé en tant que résultat de détection (C) au comparateur de valeur seuil (18) de l'unité de traitement (13), par l'intermédiaire de la liaison électrique entre les éléments de contact (6, 7) et l'aiguille électroconductrice (4a).

17. Dispositif d'administration selon l'une des revendications 1 à 7, **caractérisé en ce**
- **qu'**un revêtement (42) électroconducteur est présent sur une pièce de retenue (41) reliée à demeure à l'aiguille d'injection (4a) et
- en ce que deux éléments de détection (6, 7) sont prévus sous forme d'éléments de contact (6, 7), qui en présence d'une aiguille d'injection (4a) sont en contact électrique avec le revêtement électroconducteur,
- un signal électrique étant envoyé en tant que résultat de détection (C) au comparateur de valeur seuil (18) de l'unité de traitement (13), en particulier par l'intermédiaire de la liaison électrique entre les éléments de contact (6, 7) et le revêtement électroconducteur et/ou
- les éléments de contact (6, 7) étant disposés dans ou sur le capuchon emboîtable (16) dans la zone du moyen de retenue (4) ou dans la zone de l'aiguille d'injection (4a).

18. Procédé pour la détermination d'un paramètre physique ou chimique (P) en liaison avec un récipient (2) ou un liquide (1) se trouvant dans le récipient (2), en particulier du niveau de remplissage du liquide dans le récipient (2),
- le récipient (2) présentant à une extrémité une ouverture (3) pour l'administration du liquide (1) et un moyen de retenue (4) étant prévu dans la zone de l'ouverture (3) pour retenir une aiguille d'injection électroconductrice (4a) destinée à injecter le liquide (1) se trouvant dans le récipient (2) et
- une valeur de mesure (M) étant déterminée dans la zone du récipient (2),
**caractérisé en ce**
- **qu'**un résultat de détection (C), dépendant de la présence d'une aiguille d'injection (4a), est déterminé à l'aide d'un élément de détection (6) se trouvant dans la zone de l'ouverture (3), par détermination de la capacité entre une électrode de détection métallique (6) se trouvant dans la zone de l'ouverture (3) et une structure métallique supplémentaire (7) disposée dans ou sur le dispositif d'administration,
- en ce que le résultat de détection (C) est comparé à une valeur seuil (T) et indique en fonction de la comparaison avec la valeur seuil la présence ou l'absence d'une aiguille d'injection (4a) dans le moyen de retenue,
- lors de la détection de la présence d'une aiguille d'injection, un premier paramètre (P1) étant utilisé sur la valeur de mesure (M) par utilisation d'une première règle de calcul qui sur la base de la valeur de mesure (M) fournit la valeur du paramètre (P) en présence de l'aiguille d'injection (4a) dans le moyen de retenue (4) et la première valeur du paramètre (P1) ainsi obtenue étant mise à disposition en tant que valeur du paramètre (P) et
- lors de la détection de l'absence d'une aiguille d'injection, une deuxième valeur du paramètre (P2) étant utilisée sur la valeur de mesure (P) par utilisation d'une seconde règle de calcul qui sur la base de la valeur de mesure (M) fournit la valeur du paramètre (P) en l'absence de l'aiguille d'injection (4a) dans le moyen de retenue (4) et le second paramètre (P2) ainsi obtenu étant mis à disposition en tant que valeur du paramètre (P).

19. Procédé selon la revendication 18, **caractérisé en ce**
- **qu'**une première fonction d'étalonnage (F1) est prédéfinie, qui indique la relation entre la valeur de mesure (M) et la valeur du paramètre physique ou chimique (P), en liaison avec le récipient (2) et/ou le liquide (1) se trouvant dans ce dernier, en présence d'une aiguille d'injection dans le moyen de retenue (4),
- en ce qu'une seconde fonction d'étalonnage (F2) est prédéfinie, qui indique la relation entre la valeur de mesure (M) et la valeur du paramètre physique ou chimique (P) en relation avec le récipient (2) ou le liquide (1) se trouvant dans ce dernier, en l'absence d'aiguille d'injection dans le moyen de retenue et
- en ce que, lors de la détection de la présence d'une aiguille d'injection (4a), la première fonction d'étalonnage (F1) est utilisée sur la valeur de mesure (M) et le résultat ainsi obtenu est mis à disposition en tant que paramètre (P) et
- en ce que, lors de la détection de l'absence d'une aiguille d'injection (4a), la seconde fonction d'étalonnage (F2) est utilisée sur la valeur de mesure (M) et le résultat ainsi obtenu est mis à disposition en tant que valeur du paramètre (P).

20. Procédé selon l'une des revendications 18 ou 19, **caractérisé en ce qu'**on utilise en tant que valeur de mesure (M) la capacité pouvant être prélevée entre deux électrodes de mesure (9, 10) disposées dans la zone extérieure, s'appuyant en particulier contre la paroi du récipient (2).
